# EUROPEAN PATENT APPLICATION

(11) **EP 4 458 387 A1**
(43) Date of publication of application: **06.11.2024**
(21) Application number: 23756052.9
(22) Date of filing: 16.01.2023
(51) Int. Cl.: A61M 5/20, A61M 5/32

(54) **DRUG SOLUTION ADMINISTRATION DEVICE**

(30) Priority: 15.02.2022 JP 2022021484
(71) Applicant: Terumo Kabushiki Kaisha, Tokyo 151-0072 (JP)
(72) Inventor: MATSUMOTO, Fumiya, Fujinomiya-shi, Shizuoka 418-0004 (JP)
(74) Representative: Casalonga
(86) International application number: PCT/JP2023/000939
(87) International publication number: WO 2023/157524

(57) **Abstract**

A medicinal solution administration device (10) includes a drive unit (U) that biases a gasket (106) of a syringe (16) by a plunger (82) along with movement of a needle cover (14) in a proximal direction. The drive unit (U) includes a locking mechanism (R) that blocks movement of the plunger (82) in a distal direction in an initial state and allows the plunger (82) to move in the distal direction along with the movement of the needle cover (14) in the proximal direction. A proximal end of the needle cover (14) and the drive unit (U) are connected to each other in an axial direction by a coupling mechanism (19).

## Description

### Technical Field

The present invention relates to a medicinal solution administration device for administering a medicinal solution to a pierced living body.

### Background Art

In the related art, there is known a portable medicinal solution administration device which is used for a subcutaneous injection to pierce the skin of a user (patient), or a target of administration, and administer a medicinal solution. A medicinal solution administration device disclosed in JP 6334717 B1 includes a cylindrical casing that houses a syringe main structure, a triggering member as a needle cover slidably disposed on a distal end of the casing, a cover cap detachably disposed on the distal end of the casing, and a syringe housed in the casing and filled with a medicinal solution.

In a case where the medicinal solution administration device is accidentally dropped on the floor or the like from the cover cap side before the medicinal solution is administered by the medicinal solution administration device, the triggering member is pushed up in a direction away from the distal end due to an impact applied to the cover cap. In this instance, an arm of the triggering member comes into contact with a stopper of the casing and restricts the movement of the triggering member toward the proximal side. This configuration prevents erroneous actuation of a drive unit that drives a gasket (piston) inside the syringe in a distal direction.

### Summary of Invention

### Technical Problem

In the aforementioned medicinal solution administration device, the movement of the triggering member toward the proximal end is restricted by the stopper but the drive unit for driving the gasket is not restricted at all. For this reason, when the medicinal solution administration device is accidentally dropped on the floor or the like from the cover cap side, it is conceivable that a part of the drive unit moves inside the casing due to an inertial force and that the drive unit is actuated and pushes the piston (the drive unit actuates erroneously).

An object of the present invention is to solve the problem.

### Solution to Problem

An aspect of the present invention includes:
a casing formed into a hollow cylindrical shape;
a syringe including a cylinder that is housed in the casing and filled with a medicinal solution and a puncture needle that is communicated with the cylinder and administers the medicinal solution to a living body;
a needle cover having a hollow cylindrical shape that is disposed inside the casing and covers a distal side of the syringe and displaces in a proximal direction relative to the casing when being pressed against a puncture target;
a cap that is detachably disposed on a distal end of the needle cover and detached when the puncture target is punctured with the puncture needle; and
a drive unit that is housed on a proximal side of the casing and biases a gasket of the syringe toward a distal end of the casing by a plunger along with movement of the needle cover in the proximal direction, wherein
the drive unit includes a locking mechanism that blocks the plunger from moving in a distal direction in an initial state and allows the plunger to move in the distal direction along with the movement of the needle cover in the proximal direction, and
the medicinal solution administration device includes
a movement restricting mechanism that restricts the movement of the needle cover in the proximal direction relative to the casing when the cap is attached and allows the movement of the needle cover in the proximal direction when the cap is detached from the casing and
a coupling mechanism that couples a proximal end of the needle cover and the drive unit to each other in an axial direction.

### Advantageous Effects of Invention

According to the present invention, when the medicinal solution administration device is dropped from the cap side, the movement restricting mechanism prevents the movement of the needle cover in the proximal direction relative to the cap, and when an inertial force acts on the drive unit, the coupling mechanism is involved to prevent the drive unit from moving in the proximal direction relative to the needle cover. With this configuration, even when the medicinal solution administration device is dropped, the drive unit is not activated and the gasket is not pushed by the plunger. In other words, it is possible to reliably prevent erroneous actuation of the drive unit.

### Brief Description of Drawings

Fig. 1 is an external perspective view of a medicinal solution administration device according to an embodiment of the present invention.
Fig. 2 is an exploded perspective view of the medicinal solution administration device illustrated in Fig. 1.
Fig. 3 is an overall cross-sectional view of the medicinal solution administration device illustrated in Fig. 1.
Fig. 4 is an enlarged cross-sectional view illustrating a distal end and its neighboring parts of the medicinal solution administration device illustrated in Fig. 3.
Fig. 5 is an enlarged cross-sectional view illustrating a proximal end and its neighboring parts of the medicinal solution administration device illustrated in Fig. 3.
Fig. 6 is an overall cross-sectional view illustrating another cross section of the medicinal solution administration device illustrated in Fig. 3.
Fig. 7 is an enlarged cross-sectional view illustrating the proximal end and its neighboring parts of the medicinal solution administration device illustrated in Fig. 6.
Fig. 8 is a partially omitted external perspective view of the medicinal solution administration device illustrated in Fig. 1.
Fig. 9 is an exploded perspective view of a drive unit disassembled from a casing in the medicinal solution administration device illustrated in Fig. 8.
Fig. 10 is an enlarged perspective view illustrating a first connection and its neighboring parts of the medicinal solution administration device illustrated in Fig. 9.
Fig. 11 is an enlarged perspective view illustrating a second connection and its neighboring parts of the medicinal solution administration device illustrated in Fig. 9.
Fig. 12 is an enlarged perspective view illustrating a coupling mechanism and its neighboring parts of the medicinal solution administration device illustrated in Fig. 8.
Fig. 13 is an enlarged front view of the coupling mechanism and its neighboring parts illustrated in Fig. 12.

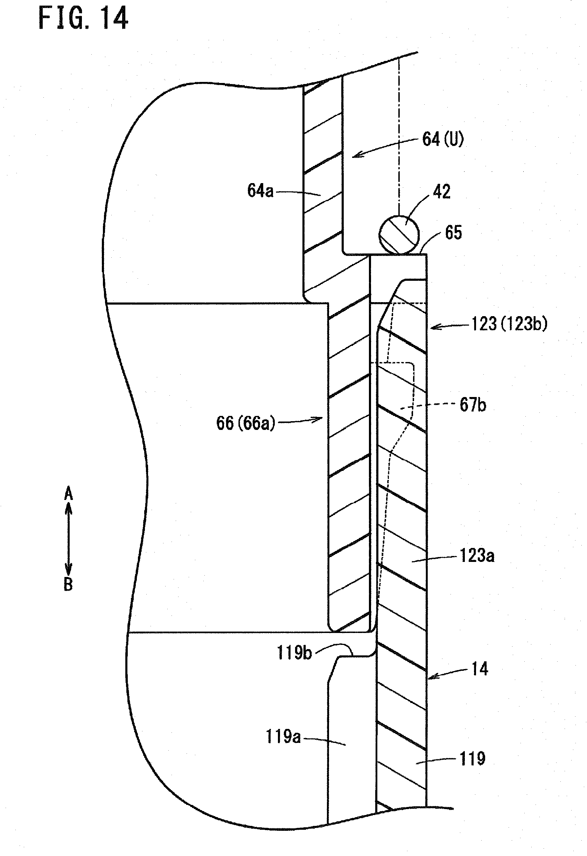

Fig. 14 is a cross-sectional view taken along line XIV-XIV in Fig. 13.
Fig. 15 is an exploded perspective view of the drive unit illustrated in Figs. 8 and 9.
Fig. 16 is an enlarged front view illustrating a state before coupling the first connection and the second connection of the coupling mechanism illustrated in Fig. 13.
Fig. 17 is an enlarged front view illustrating a state where the first connection and the second connection of the coupling mechanism illustrated in Fig. 16 are being coupled.
Fig. 18A is a partially cutaway enlarged perspective view illustrating a state where a proximal end of a plunger is inserted into a guiding groove of an end guide, and Fig. 18B is a view for illustrating an action around a distal end of the plunger illustrated in Fig. 18A.
Fig. 19A is a partially cutaway enlarged perspective view illustrating a state where the plunger starts to rotate along an inclined guiding portion, and Fig. 19B is a view for illustrating an action around the distal end of the plunger illustrated in Fig. 19A.
Fig. 20A is a partially cutaway enlarged perspective view illustrating a state where the plunger moves in a distal direction along a linear guiding portion, and Fig. 20B is a view for illustrating an action around the distal end of the plunger illustrated in Fig. 20A.
Fig. 21 is an enlarged cross-sectional view illustrating a state where a cap in the medicinal solution administration device illustrated in Fig. 4 is pulled in the distal direction.
Fig. 22 is an enlarged cross-sectional view of the cap illustrated Fig. 21, illustrating a state where a proximal end of a retaining arm of the cap tilts radially outward.
Fig. 23 is an overall cross-sectional view illustrating a state where the cap is detached from the medicinal solution administration device illustrated in Fig. 3.
Fig. 24 is an enlarged cross-sectional view illustrating the distal end and its neighboring parts of the medicinal solution administration device illustrated in Fig. 23.
Fig. 25 is an enlarged cross-sectional view illustrating the proximal end and its neighboring parts of the medicinal solution administration device illustrated in Fig. 23.
Fig. 26 is an overall cross-sectional view illustrating a state where the medicinal solution administration device illustrated in Fig. 23 starts skin puncture.
Fig. 27 is an enlarged cross-sectional view illustrating the proximal end and its neighboring parts of the medicinal solution administration device illustrated in Fig. 26.
Fig. 28 is an overall cross-sectional view illustrating a state where the medicinal solution administration device illustrated in Fig. 26 completes the skin puncture.
Fig. 29 is an enlarged cross-sectional view illustrating the proximal end and its neighboring parts of the medicinal solution administration device illustrated in Fig. 28.
Fig. 30 is an overall cross-sectional view illustrating another cross section of the medicinal solution administration device illustrated in Fig. 28 after the puncture.
Fig. 31 is an enlarged cross-sectional view illustrating the proximal end and its neighboring parts of the medicinal solution administration device illustrated in Fig. 29.
Fig. 32 is an overall cross-sectional view illustrating a state where the plunger in the medicinal solution administration device illustrated in Fig. 31 starts moving in the distal direction.
Fig. 33 is an enlarged cross-sectional view illustrating the proximal end and its neighboring parts of the medicinal solution administration device illustrated in Fig. 32.
Fig. 34 is an overall cross-sectional view illustrating a state where the plunger in the medicinal solution administration device illustrated in Fig. 28 further moves in the distal direction and completes medicinal solution administration.

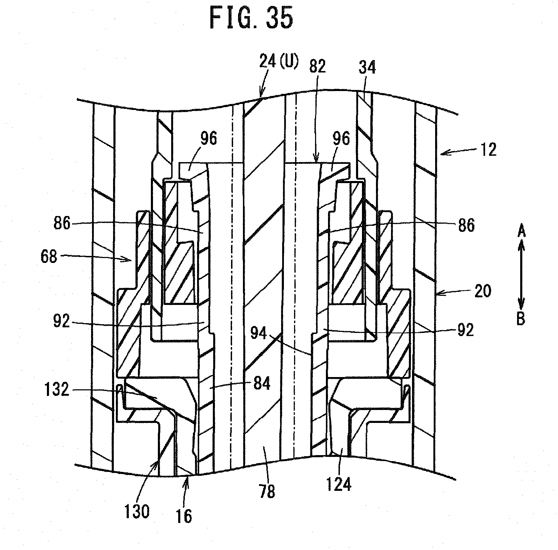

Fig. 35 is an enlarged cross-sectional view illustrating the proximal end of the plunger and its neighboring parts of the medicinal solution administration device illustrated in Fig. 34.
Fig. 36 is an overall cross-sectional view illustrating a state where the plunger in the medicinal solution administration device illustrated in Fig. 34 after the medicinal solution administration further moves in the distal direction.
Fig. 37 is an overall cross-sectional view illustrating a state where a puncture needle moves in a proximal direction and is housed in a needle cover in the medicinal solution administration device illustrated in Fig. 36.
Fig. 38 is an enlarged cross-sectional view illustrating the proximal end and its neighboring parts of the medicinal solution administration device illustrated in Fig. 37.
Fig. 39A is an enlarged front view of a medicinal solution administration device having a coupling mechanism according to a modification, and Fig. 39B is an enlarged front view illustrating a state before the coupling mechanism illustrated in Fig. 39A is coupled.
Fig. 40 is a cross-sectional view taken along line XL-XL in Fig. 39A.

### Description of Embodiments

Hereinafter, preferred embodiments of the present invention will be described in detail with reference to the accompanying drawings.

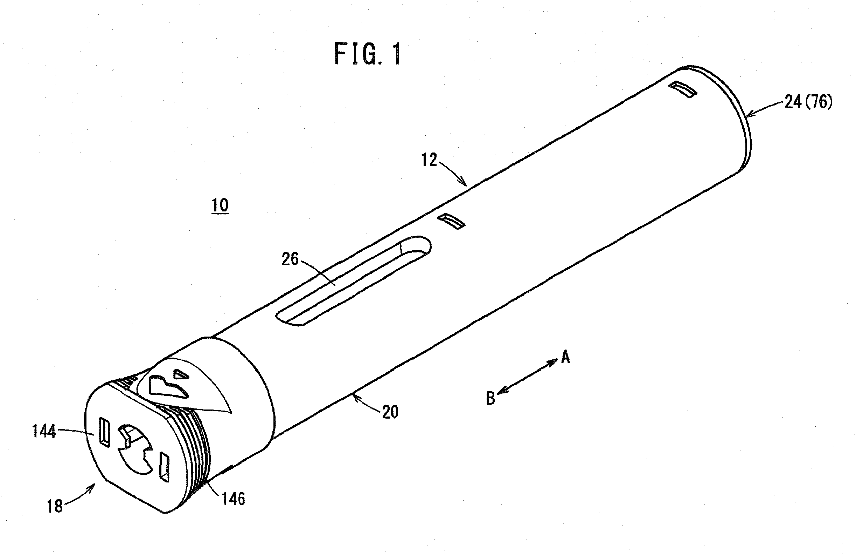

A medicinal solution administration device 10 is used, for example, for administering a medicinal solution M subcutaneously to a patient, or a user. As illustrated in Figs. 1 to 5, the medicinal solution administration device 10 includes a casing 12 formed into a hollow cylindrical shape, a needle cover 14 movably housed in the casing 12, a syringe 16 housed in the needle cover 14, a cap 18 detachably disposed on a distal end of the casing 12, and a coupling mechanism 19 that couples the needle cover 14 and a sliding member 64 to be described.

The casing 12 is formed of a resin material and has a circular cross section. The casing 12 includes a cylinder 20 having an outside diameter and an inside diameter substantially constant along the axial direction (directions of arrows A and B), a sleeve body 22 housed in the cylinder 20 on the proximal side (direction of arrow A), and an end cap 24 that closes a proximal end of the cylinder 20.

The cylinder 20 has a predetermined length in the axial direction (directions of arrows A and B). The cylinder 20 has an opened distal end and opened proximal end. The cylinder 20 has the peripheral wall provided with a viewing window 26. The viewing window 26 penetrates the peripheral wall of the cylinder 20 and is opened at a position corresponding to a position of a barrel 124 of the syringe 16 housed in the cylinder 20. The medicinal solution M in the barrel 124 can be checked through the viewing window 26.

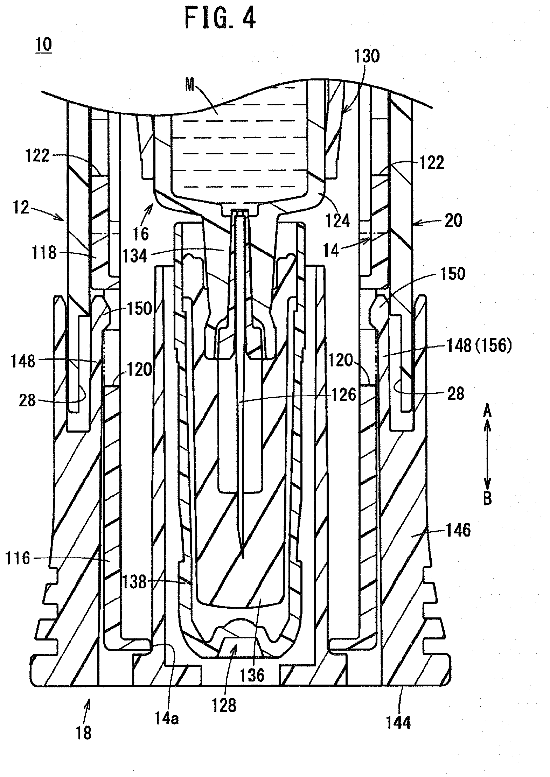

A pair of recesses 28 is formed on the distal end of the cylinder 20 (see Fig. 4). The recesses 28 have a rectangular cross section and are depressed radially with respect to the inner periphery of the cylinder 20. The pair of recesses 28 is symmetric about the axis of the cylinder 20 on the outer periphery of the cylinder 20. When the cap 18 is attached to the distal end of the cylinder 20, the recesses 28 are placed at positions facing retaining arms 148 of the cap 18.

The sleeve body 22 is formed into a hollow shape. The sleeve body 22 includes a body main structure 30 formed into a cylindrical shape and an enlarged diameter portion 32 formed on the proximal side of the body main structure 30 (direction of arrow A). The body main structure 30 has a substantially constant diameter along the axial direction (directions of arrows A and B). The body main structure 30 has the inner periphery provided with four guiding ribs 34 protruding radially inward.

The guiding ribs 34 are separated from each other at 90-degree intervals along the circumferential direction of the body main structure 30. The number of guiding ribs 34 is not limited to four. There are at least three guiding ribs 34. The guiding ribs 34 have a substantially constant thickness, protruding linearly and radially inward from the inner periphery of the body main structure 30. The guiding ribs 34 have the same radially protruding length. The guiding ribs 34 face each other in pairs across the central axis of the body main structure 30.

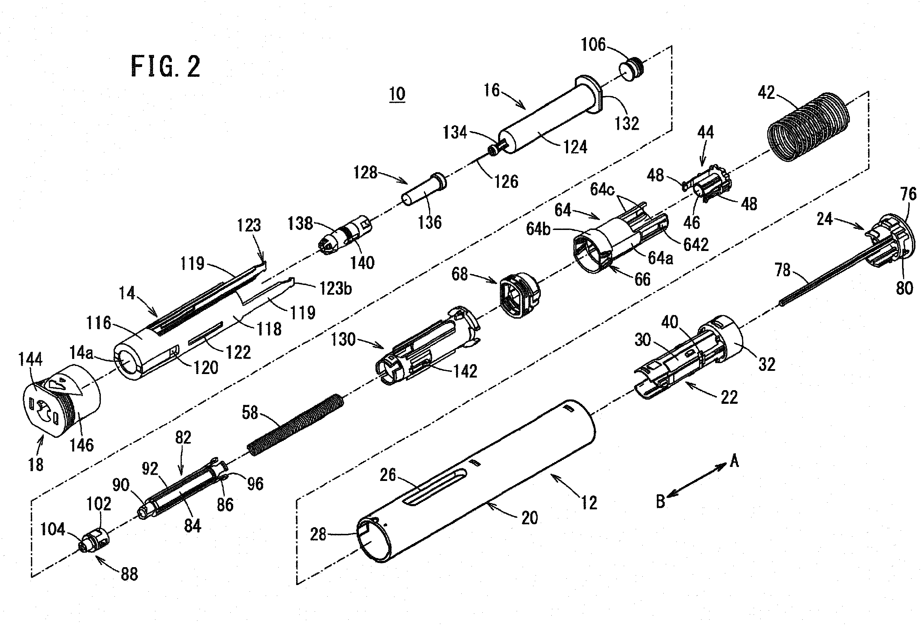

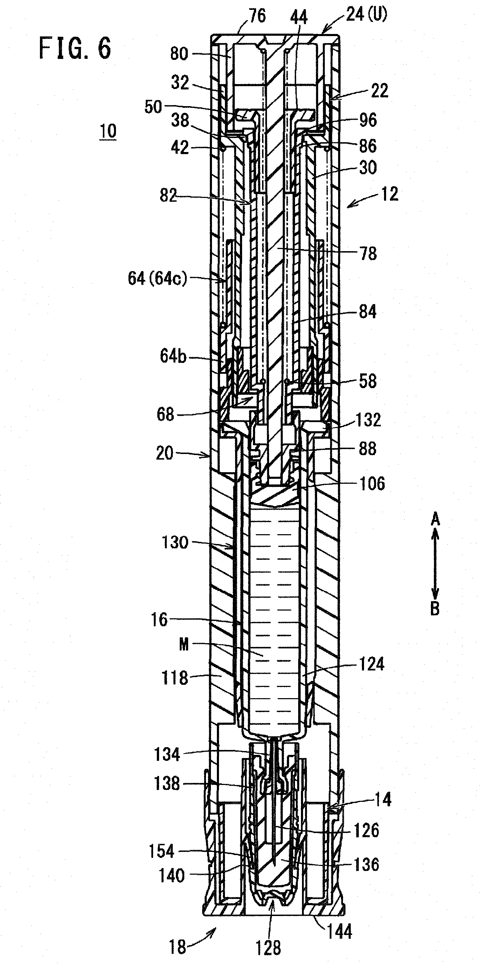

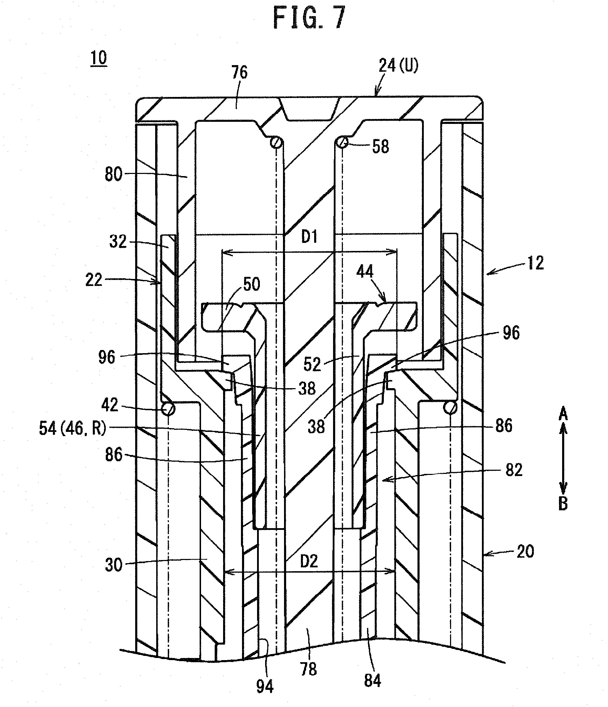

As illustrated in Figs. 2, 3, and 5, the sleeve body 22 houses a locking pin 44 and a plunger 82 to be described in a movable manner in the axial direction. As illustrated in Figs. 6 and 7, a boundary between the enlarged diameter portion 32 and the body main structure 30 is provided with a plurality of protrusions 38 protruding radially inward. The protrusions 38 have a rectangular cross section and protrude radially outward from the inner periphery of the body main structure 30. Proximal ends of the protrusions 38 are bevels that are inclined gradually and radially inward (see Fig. 7).

As illustrated in Figs. 6 and 7, the protrusions 38 are engaged with brims 96 of flexible portions 86 in the plunger 82 to be described on the proximal side (direction of arrow A). The number and arrangement of the protrusions 38 correspond to the number and arrangement of the flexible portions 86.

The sleeve body 22 is housed in the cylinder 20 from the proximal end of the cylinder 20, and the enlarged diameter portion 32 is engaged with an inner peripheral portion of the cylinder 20. Accordingly, a proximal end of the enlarged diameter portion 32 is housed and fixed at a position closer to the distal side (direction of arrow B) than the proximal end of the cylinder 20 by a predetermined distance.

As illustrated in Figs. 2, 3, and 5, the body main structure 30 has the outer periphery provided with a pair of locking grooves 40 that is depressed radially inward. The pair of locking grooves 40 is symmetric about the central axis of the sleeve body 22, and the locking grooves 40 are engaged with locking claws 62 of the locking pin 44 to be described.

A sleeve spring 42 is disposed between the outer periphery of the body main structure 30 and the inner periphery of the cylinder 20. The sleeve spring 42 is an elastic member composed of a coil spring. A proximal end of the sleeve spring 42 is engaged with a distal end of the enlarged diameter portion 32. A distal end of the sleeve spring 42 is engaged with the sliding member 64 to be described. An elastic force of the sleeve spring 42 biases the sliding member 64 in a direction away from the sleeve body 22, that is, in the distal direction (direction of arrow B).

The sleeve body 22 houses the locking pin 44 that straddles the enlarged diameter portion 32 and the body main structure 30.

The locking pin 44 is movable in the axial direction (directions of arrows A and B) inside the sleeve body 22. The locking pin 44 includes a cylindrical pin main structure 46 formed at the center and a pair of arms 48 disposed on the outer periphery of the pin main structure 46. The arms 48 are connected to a proximal end of the pin main structure 46 in an integrated manner.

The pin main structure 46 includes a flat end wall 50 formed on the proximal end and perpendicular to the axial direction, a large diameter portion 52 extending from the center of the end wall 50 toward the distal side (direction of arrow B), and a small diameter portion 54 formed on the distal side of the large diameter portion 52 (direction of arrow B) with a diameter smaller than that of the large diameter portion 52. The pin main structure 46 has a hole 56 on the central axis. The hole 56 extends along the axial direction with a constant diameter, linearly penetrating the end wall 50, the large diameter portion 52, and the small diameter portion 54. Into the hole 56, a shaft 78 of the end cap 24 and an injection spring 58 to be described are inserted.

Proximal ends of the arms 48 are connected to the end wall 50 of the pin main structure 46 and are arranged in parallel at a predetermined interval on the outer periphery of the pin main structure 46. The arms 48 have the same length, extending toward the distal end (direction of arrow B). Distal ends of the arms 48 have engaging ends 60 engaged with the sliding member 64 to be described and the locking claws 62 formed on the radially inner side of the engaging ends 60.

The engaging ends 60 have a flat shape perpendicular to a direction of extension of the arms 48. The locking claws 62 protrude radially inward from the engaging ends 60 and toward the distal side (direction of arrow B). The locking claws 62 have a triangular cross-sectional shape tapered toward the distal ends of the arms 48.

Centers of the arms 48 in the longitudinal direction are provided with protrusive portions 48a that protrude outward. The protrusive portions 48a protrude outward from outer surfaces of the arms 48 and have a rectangular shape elongated in a direction perpendicular to the direction of extension of the arms 48. The protrusive portions 48a are inserted into slits 641 of the sliding member 64 to be described.

Inside the cylinder 20, the cylindrical sliding member 64 is disposed on the distal side (direction of arrow B) of the locking pin 44.

The sliding member 64 is movable in the axial direction (directions of arrows A and B) inside the cylinder 20. The plunger 82 and the body main structure 30 of the sleeve body 22 are inserted into the sliding member 64.

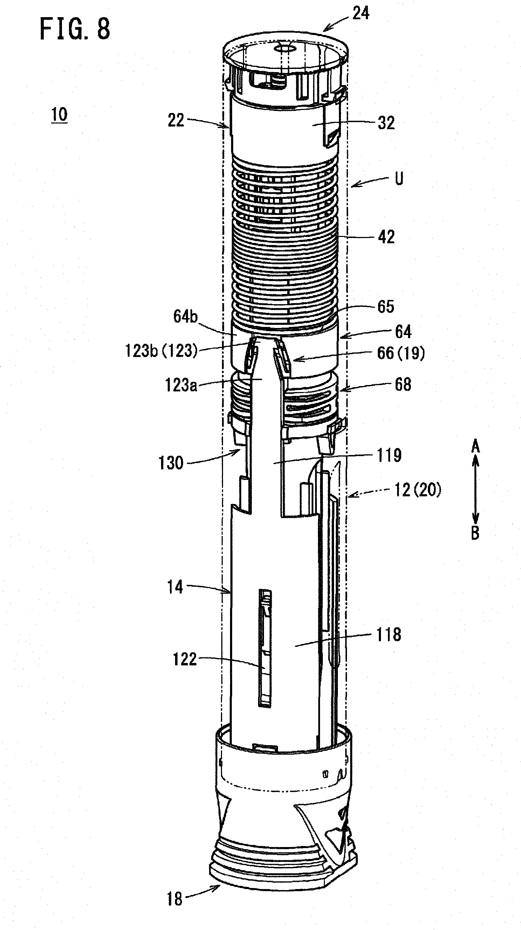

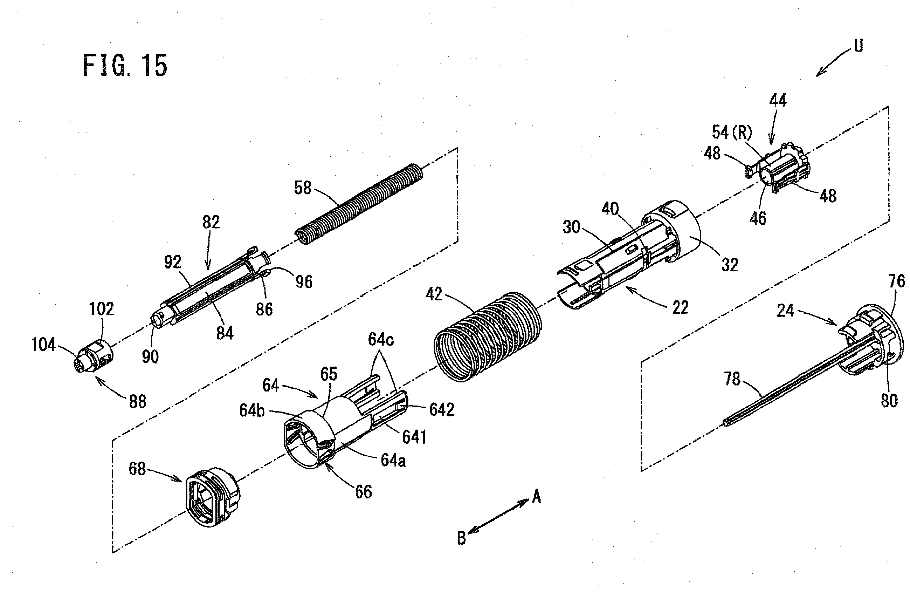

As illustrated in Fig. 15, the sliding member 64 includes a cylindrical sleeve main structure 64a, a cylindrical portion 64b disposed on a distal end of the sleeve main structure 64a, and a pair of engaging arms 64c extending from a proximal end of the sleeve main structure 64a. The cylindrical portion 64b increases in diameter as compared with the sleeve main structure 64a. That is, the diameter of the cylindrical portion 64b is larger than that of the sleeve main structure 64a. A boundary between the sleeve main structure 64a and the cylindrical portion 64b has a stepped catcher 65. As illustrated in Fig. 8, the catcher 65 retains the distal end of the sleeve spring 42 disposed on the outer periphery of the sliding member 64. An elastic force of the sleeve spring 42 biases the sliding member 64 toward the distal end.

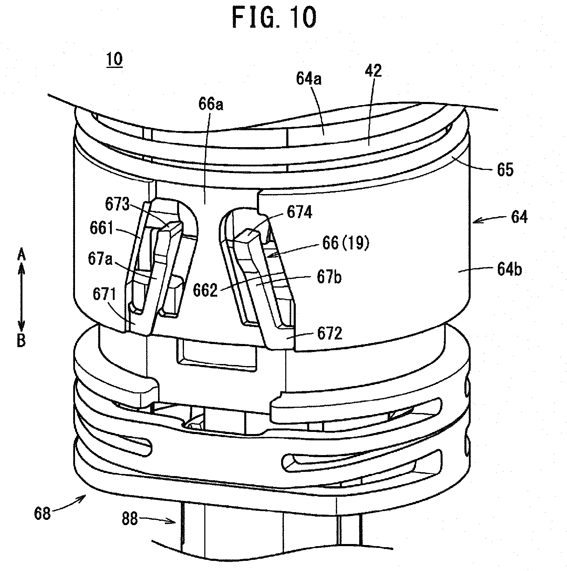

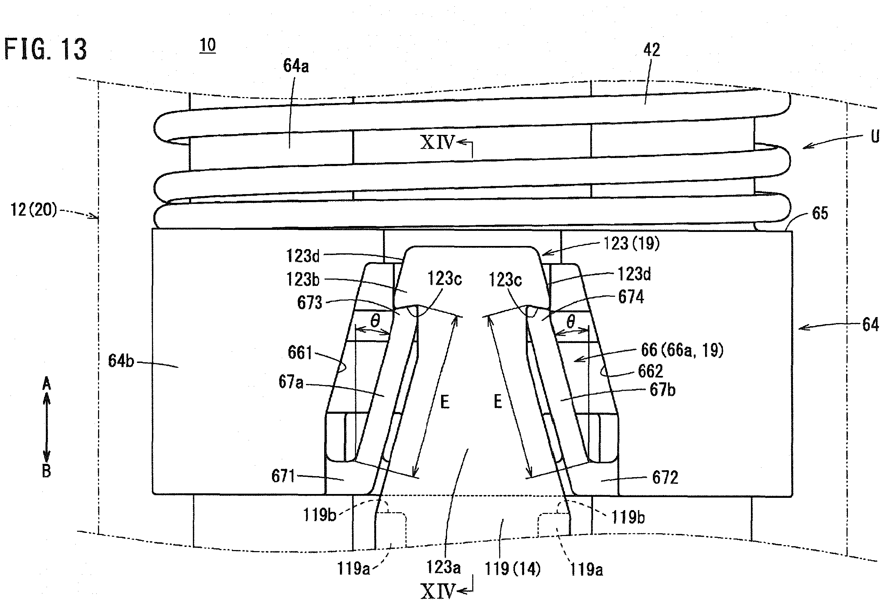

As illustrated in Fig. 10, the cylindrical portion 64b has first connections 66 as part of the coupling mechanism 19. The first connections 66 are disposed in pairs, being apart from each other along the circumferential direction of the cylindrical portion 64b. The first connections 66 are symmetric about the axis of the sliding member 64. Each first connection 66 has a housing recess 66a and first and second flexible arm portions 67a and 67b disposed in the housing recess 66a. Each housing recess 66a is depressed radially inward from the outer periphery of the cylindrical portion 64b and is open from a distal end of the cylindrical portion 64b toward a proximal end. As illustrated in Fig. 13, each housing recess 66a has a trapezoidal shape narrowed toward a proximal end of the sliding member 64. In other words, side walls 661 and 662 constituting both sides of each housing recess 66a in the circumferential direction of the cylindrical portion 64b are inclined inward from the distal end toward the proximal end.

The first and second flexible arm portions 67a and 67b are separated from each other along the circumferential direction of the cylindrical portion 64b and are respectively arranged along the inclined side walls 661 and 662 of each housing recess 66a. The first flexible arm portion 67a and the second flexible arm portion 67b are symmetrical about the center of each housing recess 66a. The first and second flexible arm portions 67a and 67b are in parallel to the side walls 661 and 662 of each housing recess 66a while being separated from the side walls 661 and 662.

One ends (ends in the direction of arrow B) of the first and second flexible arm portions 67a and 67b are fixed ends 671 and 672 and are connected to the distal end of the cylindrical portion 64b. The first and second flexible arm portions 67a and 67b are inclined at an angle 8, having the fixed ends 671 and 672 of the first and second flexible arm portions 67a and 67b functioning as bases. The angle θ is, for example, in the range of 5 to 45 degrees. The other ends (end in the direction of arrow A) of the first and second flexible arm portions 67a and 67b are free ends 673 and 674 and are not connected to the cylindrical portion 64b. The fixed ends 671 and 672 of the first and second flexible arm portions 67a and 67b are closer to the distal end of the cylindrical portion 64b than the free ends 673 and 674.

The first and second flexible arm portions 67a and 67b have a tiltable cantilever structure with the fixed ends 671 and 672 functioning as bases. The first and second flexible arm portions 67a and 67b are elastically deformable. A length E from the fixed ends 671 and 672 to the free ends 673 and 674 of the first and second flexible arm portions 67a and 67b is, for example, 3 mm or more along a direction of extension of the first and second flexible arm portions 67a and 67b. The first flexible arm portion 67a and the second flexible arm portion 67b have the same length.

Each housing recess 66a has a space between the first flexible arm portion 67a and the second flexible arm portion 67b. Each space houses a second connection 123 of the needle cover 14, that is, part of the coupling mechanism 19.

As illustrated in Fig. 15, in the sliding member 64, the pair of engaging arms 64c is symmetric about the axis of the sliding member 64, and each engaging arm 64c protrudes in the proximal direction of the sliding member 64. The engaging arms 64c have the slits 641. The slits 641 have a rectangular shape along a direction of extension of the engaging arms 64c. Proximal ends of the slits 641 have protruding pieces 642 protruding from proximal ends of the engaging arms 64c toward distal ends. The protruding pieces 642 are inclined inward toward the distal ends.

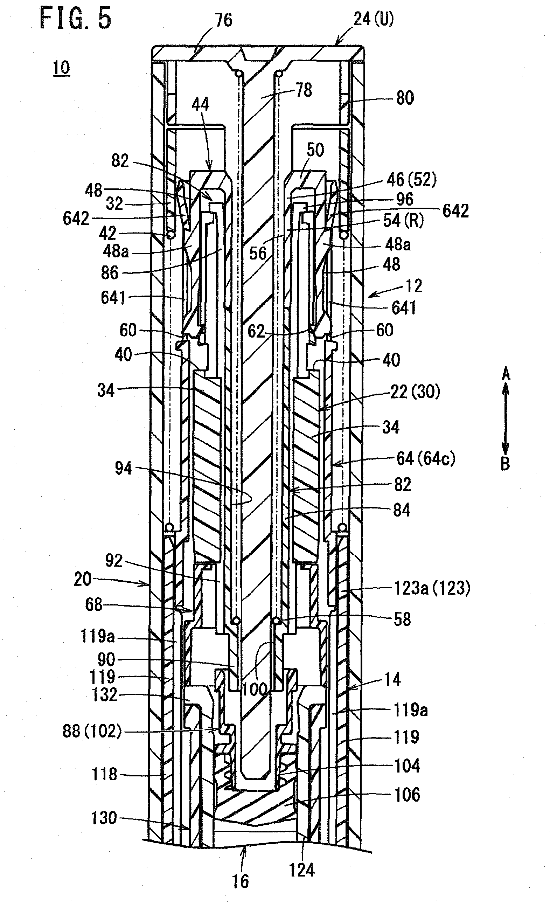

As illustrated in Fig. 5, a part of each arm 48 of the locking pin 44 is inserted into each slit 641. The engaging ends 60 of the locking pin 44 can be engaged with distal ends of the slits 641. The protrusive portions 48a of the arms 48 can be engaged with the protruding pieces 642. Accordingly, the locking pin 44 is engaged with the sliding member 64, and the locking pin 44 is prevented from being detached from the sliding member 64 in the proximal direction.

As illustrated in Fig. 8, an end guide 68 is disposed to face a distal end of the sliding member 64. The end guide 68 has a cylindrical shape having a diameter substantially equal to that of the sliding member 64. As illustrated in Fig. 5, the plunger 82 is inserted into the end guide 68 in a movable manner in the axial direction.

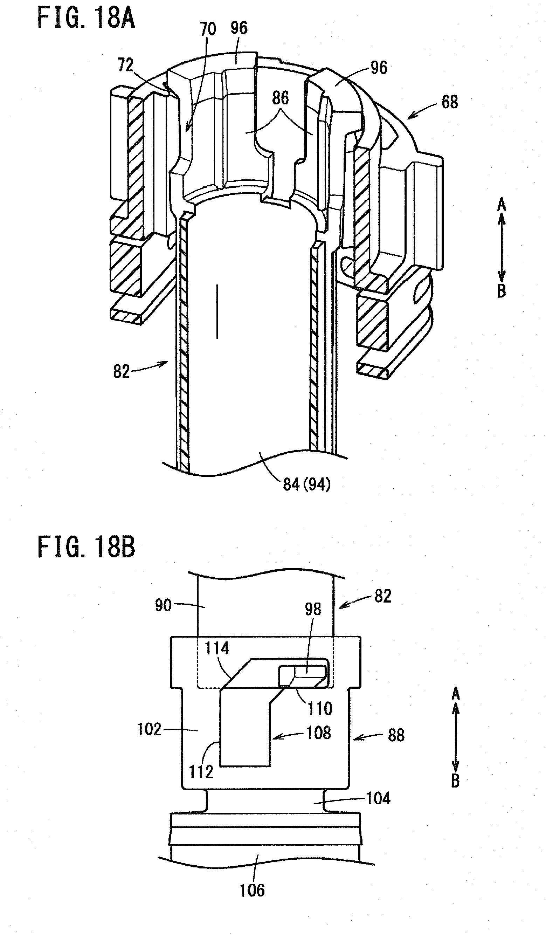

As illustrated in Fig. 18A, guiding grooves 70 for rotating the plunger 82 are disposed on the inner periphery of the end guide 68. The guiding grooves 70 are depressed radially outward with respect to the inner periphery of the end guide 68. The brims 96 of the flexible portions 86 of the plunger 82 to be described are inserted into the guiding grooves 70. The guiding grooves 70 are arranged at four places at equal intervals in the circumferential direction, corresponding to the number and arrangement of the flexible portions 86.

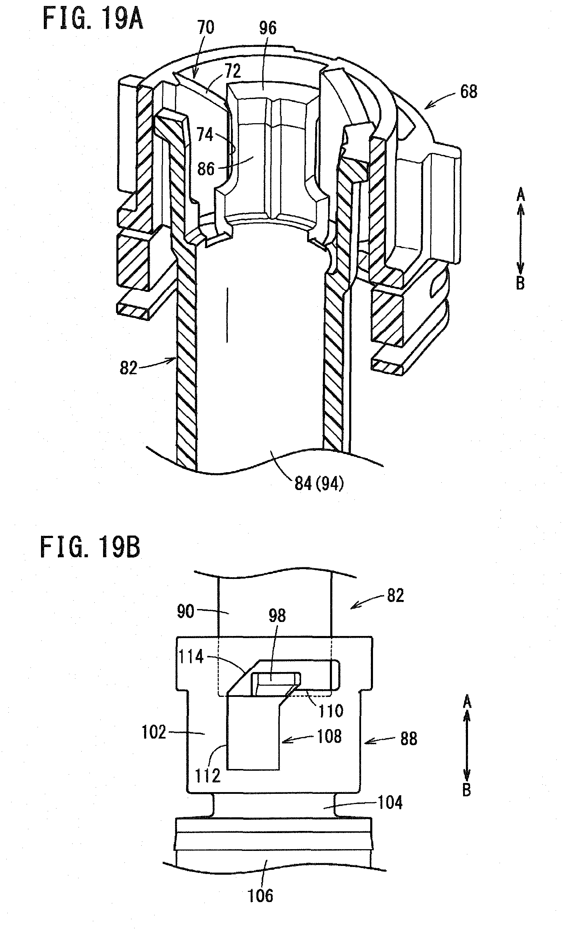

As illustrated in Fig. 19A, each guiding groove 70 includes an inclined guiding portion 72 and a linear guiding portion 74. The inclined guiding portions 72 are inclined in the distal direction (direction of arrow B) with respect to the axial direction (directions of arrows A and B) of the end guide 68. The linear guiding portions 74 extend in a straight line from distal ends of the inclined guiding portions 72. A width along the circumferential direction of the linear guiding portions 74 is substantially equal to or slightly larger than a width of the brims 96 of the plunger 82 to be described.

As illustrated in Fig. 5, the end cap 24 has a lid 76 that closes the proximal end of the cylinder 20 and the shaft 78 extending from the center of the lid 76 toward the distal side (direction of arrow B). The lid 76 has a disk-like shape having the same diameter as the outside diameter of the cylinder 20.

The lid 76 has a pusher 80 protruding from an end face on the distal side. The pusher 80 is formed into an annular shape protruding from the end face of the lid 76. When the pusher 80 abuts on the proximal end of the enlarged diameter portion 32 of the sleeve body 22, the sleeve body 22 housed in the cylinder 20 is restricted from moving in the proximal direction (direction of arrow A) and retained.

The shaft 78 is a shaft body extending toward the distal side (direction of arrow B) along the axial direction. The shaft 78 is housed in the cylinder 20 and the sleeve body 22, extending to a part near the center of the cylinder 20 in the axial direction. The injection spring 58 and the plunger 82 are allowed to pass through the outer periphery of the shaft 78. The injection spring 58 is a coil spring, having an elongated shape corresponding to the axial length of the shaft 78. The injection spring 58 is interposed between the plunger 82 to be described and the end face of the lid 76, and an elastic force of the injection spring 58 biases the plunger 82 in the distal direction (direction of arrow B).

When the end cap 24 is attached to the proximal end of the cylinder 20, the opened proximal end is closed by the lid 76 and the shaft 78 is disposed on the axis of the cylinder 20.

As illustrated in Figs. 2 and 5, the plunger 82 has a cylindrical shape elongated in the axial direction (directions of arrows A and B). The plunger 82 includes a rod main structure 84 having a constant diameter along the axial direction, a plurality of flexible portions 86 formed on the proximal side (direction of arrow A) of the rod main structure 84 and divided in the circumferential direction, and an attaching portion 90 formed on the distal side (direction of arrow B) of the rod main structure 84 and to which a top 88 to be described is attached.

The rod main structure 84 has a circular cross section. As illustrated in Fig. 2, the rod main structure 84 has the outer periphery provided with four sliding grooves 92 that are depressed radially inward. The sliding grooves 92 have a rectangular cross section and are depressed with respect to the outer periphery of the rod main structure 84. As illustrated in Fig. 5, the guiding ribs 34 of the sleeve body 22 are inserted into the sliding grooves 92. The sliding grooves 92 are separated at equal intervals in the circumferential direction, corresponding to the number and arrangement of the guiding ribs 34. The sliding grooves 92 correspond to the four guiding ribs 34.

When the plunger 82 is housed in the sleeve body 22, distal ends of the guiding ribs 34 are inserted into the sliding grooves 92.

The inside of the rod main structure 84 has a first rod hole 94 extending along the axial direction (directions of arrows A and B). Into this first rod hole 94, the shaft 78 of the end cap 24 and the injection spring 58 are inserted.

The flexible portions 86 protrude in the axial direction (direction of arrow A) from a proximal end of the rod main structure 84 and are separated from each other in the circumferential direction. Each flexible portion 86 is disposed between two adjacent sliding grooves 92. The distal side (direction of arrow B) of the flexible portions 86 connected to the rod main structure 84 functions as a fulcrum, and the proximal side (direction of arrow A) of the flexible portions 86 is radially tiltable. As illustrated in Fig. 7, proximal ends of the flexible portions 86 are provided with the brims 96 protruding radially outward. Surfaces of the brims 96 on the distal side (direction of arrow B) are bevels that are inclined gradually and radially outward toward the proximal side.

The flexible portions 86 have a diameter increasing radially outward from the rod main structure 84. The small diameter portion 54 of the locking pin 44 is inserted into the interior of the flexible portions 86. The inner peripheral diameter of the flexible portions 86 is larger than that of the first rod hole 94.

As illustrated in Fig. 7, the brims 96 of the flexible portions 86 have an outside diameter D1 slightly larger than an inside diameter D2 of the body main structure 30 of the sleeve body 22 (D1 > D2).

As illustrated in Fig. 15, the attaching portion 90 has a small diameter with respect to the rod main structure 84. The outer periphery of the attaching portion 90 is provided with a pair of engaging chips 98 protruding radially outward. The pair of engaging chips 98 is engaged with the top 88 to be described. The engaging chips 98 extend in the horizontal direction perpendicular to the axial direction of the plunger 82, being symmetric about the central axis of the plunger 82.

As illustrated in Fig. 5, the attaching portion 90 includes a second rod hole 100 extending along the axial direction (directions of arrows A and B). The second rod hole 100 is smaller in diameter than the first rod hole 94, and the shaft 78 of the end cap 24 is inserted through the second rod hole 100. A distal end of the injection spring 58 is engaged with a stepped portion formed at a boundary between the second rod hole 100 and the first rod hole 94. Accordingly, an elastic force of the injection spring 58 biases the plunger 82 in a direction away from the end cap 24, that is, in the distal direction (direction of arrow B) .

As illustrated in Fig. 5, the top 88 has a cup-like shape with the proximal side (direction of arrow A) opened. The top 88 is rotatable relative to the attaching portion 90 of the plunger 82. The top 88 includes a cylindrical cup portion 102 disposed on the proximal side and a mounting portion 104 protruding from a distal end of the cup portion 102. A gasket 106 formed of an elastic material is attached to the top 88 so as to cover the outer periphery of the mounting portion 104.

The attaching portion 90 of the plunger 82 is inserted into the cup portion 102 from the proximal side. As illustrated in Fig. 18B, the outer periphery of the cup portion 102 is provided with a pair of introducing grooves 108 which penetrates the cup portion 102 radially and through which the engaging chips 98 of the plunger 82 are inserted.

The introducing grooves 108 are arranged in pairs, being symmetric about the central axis of the cup portion 102. Each introducing groove 108 includes a horizontal portion 110 formed near a proximal end of the cup portion 102 and extending along the circumferential direction and a perpendicular portion 112 perpendicular to the horizontal portion 110 and extending from one end in the circumferential direction toward the distal side (direction of arrow B). Each introducing groove 108 substantially has a L-shape including the horizontal portion 110 and the perpendicular portion 112. Each connecting portion between the horizontal portion 110 and the perpendicular portion 112 has an inclined portion 114 inclined at a predetermined angle.

In the top 88, the attaching portion 90 of the plunger 82 is inserted into the cup portion 102, and the engaging chips 98 are inserted into the pair of introducing grooves 108. Accordingly, the top 88 and the plunger 82 are connected in the axial direction and movable along the axial direction in an integrated manner. After the gasket 106 is attached to the mounting portion 104 of the top 88, the gasket 106 is inserted into the barrel 124 of the syringe 16 to be described.

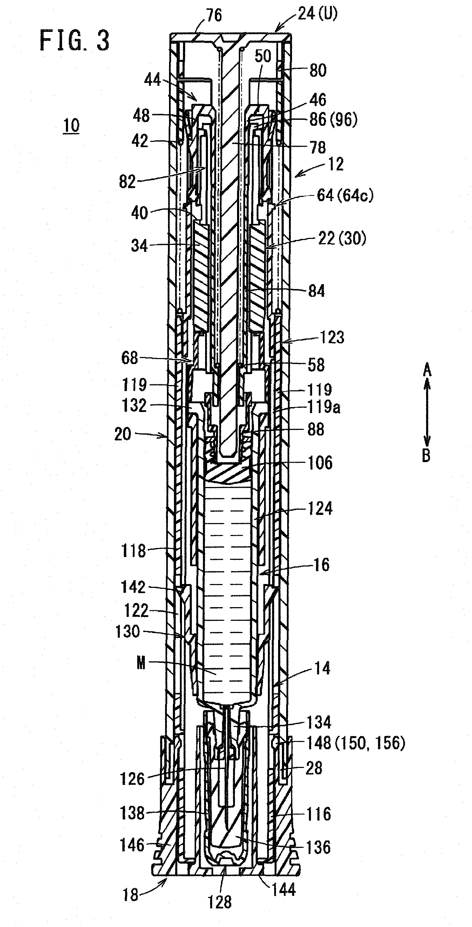

As illustrated in Fig. 3, the needle cover 14 is movably disposed inside the cylinder 20 constituting the casing 12. As illustrated in Fig. 2, the needle cover 14 includes a cylindrical main structure 116, a pair of covering portions 118 extending from the main structure 116 toward the proximal side (direction of arrow A), and a pair of extending portions 119 extending from proximal ends of the covering portions 118.

The main structure 116 is disposed on the distal side (direction of arrow B) of the needle cover 14, and the center of the main structure 116 is opened by a hole 14a penetrating the main structure 116 in the axial direction. The covering portions 118 are symmetric about the axis of the needle cover 14, extending along the axial direction.

The main structure 116 includes a pair of engaging holes 120 with which the retaining arms 148 of the cap 18 are engaged. The covering portions 118 include syringe guiding holes 122 elongated along the axial direction. The syringe guiding holes 122 and the engaging holes 120 are linearly arranged along the axial direction of the needle cover 14. The syringe guiding holes 122 and the engaging holes 120 are separated from each other by a predetermined distance in the axial direction of the needle cover 14.

The engaging holes 120 radially penetrate the main structure 116. The engaging holes 120 have a rectangular shape elongated in a direction perpendicular to the axial direction of the needle cover 14. The syringe guiding holes 122 radially penetrate the covering portions 118. A syringe holder 130 to be described is engaged with the syringe guiding holes 122 in a movable manner in the axial direction. The syringe guiding holes 122 have a function of introducing the syringe holder 130 in the axial direction.

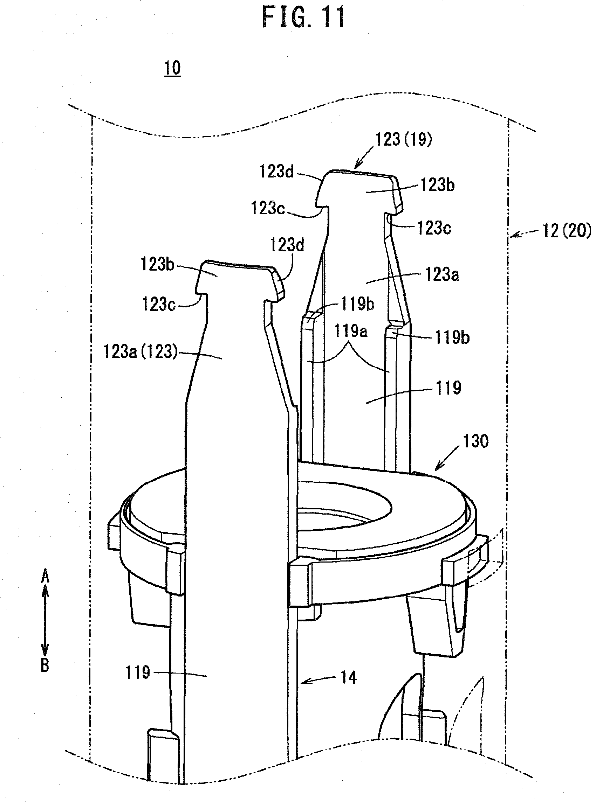

The extending portions 119 further extend from centers of the proximal ends of the covering portions 118 toward the proximal end. The extending portions 119 extend linearly with a substantially constant width. Proximal ends of the extending portions 119 include the second connections 123 of the coupling mechanism 19. As illustrated in Fig. 11, each extending portion 119 includes a pair of ribs 119a. The ribs 119a are disposed on both ends of each extending portion 119 in the width direction, extending along each extending portion 119. The ribs 119a protrude from the inner surface of each extending portion 119. Proximal ends 119b of the ribs 119a are disposed near the proximal end of each extending portion 119.

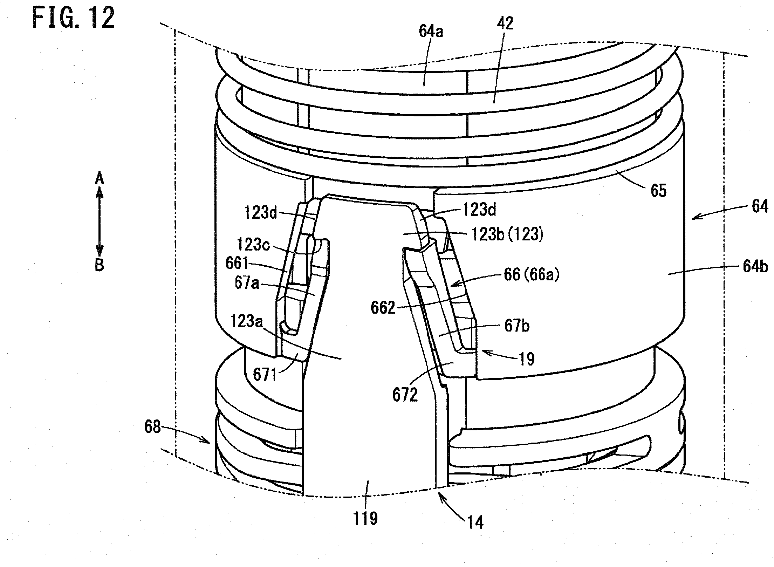

The second connections 123 are part of the coupling mechanism 19. As shown in Fig. 12, the second connections 123 can be coupled to the first connections 66 of the sliding member 64. The coupling mechanism 19 has the first and second connections 66 and 123, being capable of coupling the distal end of the sliding member 64 and a proximal end of the needle cover 14.

The second connections 123 each include an extending end 123a gradually narrowing with respect to each extending portion 119, a projection 123b disposed on a proximal end of the extending end 123a, and jaws 123c disposed on a connecting portion between the extending end 123a and the projection 123b.

The extending end 123a is inclined, gradually narrowing from the proximal end of each extending portion 119 toward the proximal direction (direction of arrow A). Side portions of the extending end 123a in the width direction are inclined.

As illustrated in Fig. 13, the side portions of the extending end 123a in the width direction and the first and second flexible arm portions 67a and 67b are substantially parallel to each other. In other words, an inclination angle of the side portions of the extending end 123a in the width direction is substantially equal to the angle θ of the first and second flexible arm portions 67a and 67b.

The projection 123b protrudes to both sides in the width direction perpendicular to a direction of extension of the extending end 123a and each extending portion 119. The projection 123b is wider than the extending end 123a in the width direction, or the circumferential direction of the cylindrical portion 64b. A maximum width of the projection 123b is substantially equal to or smaller than a width between the first flexible arm portion 67a and the second flexible arm portion 67b on the distal side. A maximum width of the projection 123b is larger than a width between the first flexible arm portion 67a and the second flexible arm portion 67b on the proximal side. Both ends of the projection 123b in the width direction have bevels 123d. The bevels 123d are inclined toward the center in the width direction of the projection 123b in a direction away from the extending end 123a (proximal direction).

Each jaw 123c is disposed on a connecting portion between a distal end of the projection 123b and the proximal end of the extending end 123a. The jaws 123c are depressed inward in the width direction from ends of the projection 123b in the width direction. Each second connection 123 substantially has a T-shape formed by the extending end 123a and the projection 123b.

As illustrated in Fig. 2, the syringe 16 includes the hollow barrel 124, the gasket 106 slidably inserted into the barrel 124, a puncture needle 126 disposed on a distal end of the barrel 124 and protruding in the distal direction (direction of arrow B), and a protective cover 128 attached to the distal end of the barrel 124.

As illustrated in Fig. 3, the cylindrical syringe holder 130 is disposed on the outer periphery of the barrel 124, and the barrel 124 is retained by the syringe holder 130. Examples of the medicinal solution M to be used include those used for subcutaneous injection to a patient.

The barrel 124 has a substantially cylindrical hollow body, having an opening at a proximal end. The barrel 124 is filled with the medicinal solution M. An outer peripheral portion on the proximal end of the barrel 124 is provided with a flange 132 protruding radially outward. A needle retainer 134 is disposed on the distal end of the barrel 124. The needle retainer 134 is reduced in diameter with respect to the barrel 124 and protrudes in the distal direction. The needle retainer 134 retains a proximal end of the puncture needle 126.

The barrel 124 is formed of a transparent resin material, and it is possible to visually check an amount of the medicinal solution M remaining inside the barrel 124 from the outside through the viewing window 26 of the casing 12. The flange 132 of the barrel 124 is engaged with a proximal end of the syringe holder 130. Accordingly, the syringe 16 does not move relative to the syringe holder 130 in the axial direction, and the outer periphery of the syringe 16 is covered and retained by the syringe holder 130 in an integrated manner.

The gasket 106 is formed of an elastic material such as rubber. The gasket 106 is attached to the mounting portion 104 of the top 88, and the gasket 106 is inserted into the barrel 124 through the proximal opening of the barrel 124. The gasket 106 is slidably housed in the axial direction along the inner periphery of the barrel 124. When the gasket 106 is inserted into the barrel 124, the proximal side of the barrel 124 is sealed in a liquid-tight manner, and the medicinal solution M is enclosed in the barrel 124.

The puncture needle 126 is a hollow body having a channel through which the medicinal solution M flows. The puncture needle 126 protrudes from the needle retainer 134 in the distal direction. The channel of the puncture needle 126 is communicated with the inside of the barrel 124 filled with the medicinal solution M. The medicinal solution M filled in the barrel 124 is ejected from a distal end of the puncture needle 126 and administered to the patient.

The protective cover 128 is attached to the distal end of the barrel 124 to cover the puncture needle 126. The protective cover 128 includes a needle shield 136 formed of an elastic material such as rubber and attached to the needle retainer 134 and an outer cover 138 that covers the outer periphery of the needle shield 136. A proximal end of the needle shield 136 is attached to the needle retainer 134 to cover the puncture needle 126. The outer cover 138 is slidably fitted into the outer periphery of the needle shield 136. As illustrated in Fig. 6, the outer periphery of the outer cover 138 is provided with an annular groove 140 that is depressed radially inward. An inner hook 154 of the cap 18 to be described is engaged with the annular groove 140.

As illustrated in Figs. 2 and 3, the syringe holder 130 includes a pair of introducing portions 142 protruding radially outward from the outer periphery. The introducing portions 142 are respectively inserted into the syringe guiding holes 122 of the needle cover 14. Accordingly, the syringe holder 130 is retained inside the needle cover 14 together with the syringe 16 in a movable manner along the axial direction (directions of arrows A and B).

When the syringe 16 is retained by the syringe holder 130 and housed in the needle cover 14, the puncture needle 126 is disposed on the distal side (direction of arrow B), and the barrel 124 faces the viewing window 26 (see Fig. 1) opened on the casing 12.

As illustrated in Fig. 4, the cap 18 has a bottomed cylindrical shape, having a distal end provided with a bottom wall 144 and an annular peripheral wall 146 erected from the bottom wall 144 toward the proximal side (direction of arrow A). A proximal end of the cap 18 is opened. The cap 18 includes a pair of retaining arms 148 protruding in the axial direction (direction of arrow A) from a proximal end of the peripheral wall 146.

The retaining arms 148 are disposed radially inward with respect to the peripheral wall 146 and are radially tiltable, having connecting portions with the peripheral wall 146 as fulcrums. Proximal ends of the retaining arms 148 are provided with outer hooks 150 protruding radially inward. The retaining arms 148 are arranged in pairs and symmetric about the central axis of the cap 18.

When the cap 18 is attached to the distal end of the casing 12, the proximal end of the peripheral wall 146 abuts on the distal end of the cylinder 20, and the pair of retaining arms 148 is inserted into gaps between the cylinder 20 and the needle cover 14. The pair of retaining arms 148 is disposed at positions facing the recesses 28 and the engaging holes 120, and the outer hooks 150 are engaged with the engaging holes 120.

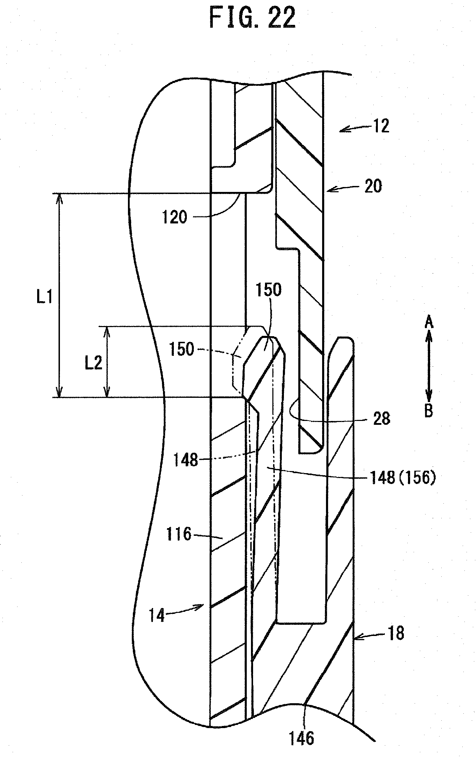

As shown in Fig. 22, the engaging holes 120 have an axial length L1 larger than an axial length L2 of the outer hooks 150 (L1 > L2). The outer hooks 150 are engaged with the engaging holes 120 in a movable manner in the axial direction (directions of arrows A and B).

When the cap 18 is attached to a distal end of the needle cover 14 illustrated in Fig. 4, the recesses 28 are offset and closer to the distal side (direction of arrow B) than the engaging holes 120. The proximal ends of the retaining arms 148 having the outer hooks 150 become closer to the proximal side (direction of arrow A) than proximal ends of the recesses 28.

As illustrated in Fig. 6, the inner hook 154 protruding radially inward is disposed inside the cap 18. The outer cover 138 of the protective cover 128 is inserted into the inner hook 154. The inner hook 154 is engaged with the annular groove 140 of the outer cover 138.

Accordingly, the cap 18 is engaged with the protective cover 128 through the inner hook 154 and is engaged with the distal end of the needle cover 14. In a state where the cap 18 retains the protective cover 128, the distal ends of the needle cover 14 and the casing 12 are covered by the cap 18 attached.

Hereinafter described is the assembly of the medicinal solution administration device 10.

First, the end cap 24, the injection spring 58, the plunger 82, the top 88, the end guide 68, the sleeve spring 42, the locking pin 44, the sleeve body 22, and the sliding member 64 illustrated in Figs. 8, 9, and 15 are assembled to constitute a drive unit U. When the medicinal solution is administered by the medicinal solution administration device 10, in an initial state where the puncture needle 126 is covered with the needle cover 14, the drive unit U has a locking mechanism R that blocks the movement of the plunger 82 in the distal direction caused by the injection spring 58. When the needle cover 14 is pressed and moved in the proximal direction, the locked state by the locking mechanism R is released, thereby allowing the plunger 82 to move in the distal direction.

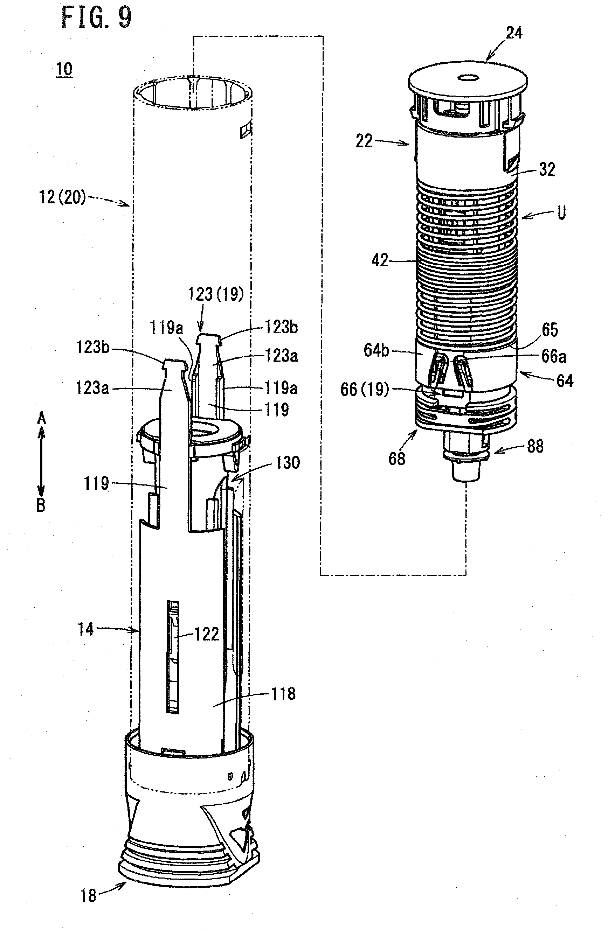

The end cap 24 is disposed on a proximal end of the drive unit U, and the end guide 68 and the top 88 are disposed on a distal end of the drive unit U (see Fig. 9). The first connections 66 of the sliding member 64 are disposed adjacent to the end guide 68.

Next, the drive unit U illustrated in Fig. 9 is inserted into the casing 12 from its proximal end. In this instance, the drive unit U is inserted in such a manner that the first connections 66 and the second connections 123 of the coupling mechanism 19 are in a straight line along the axial direction (directions of arrows A and B).

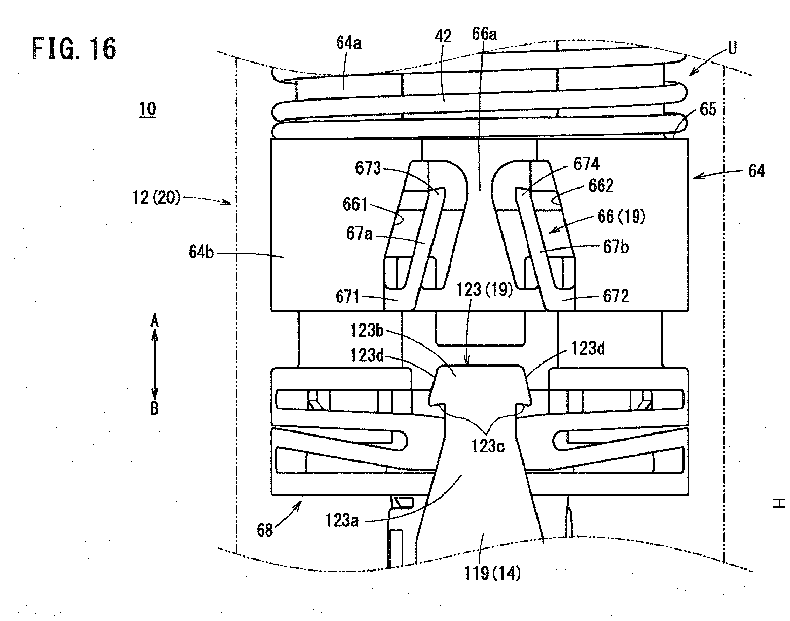

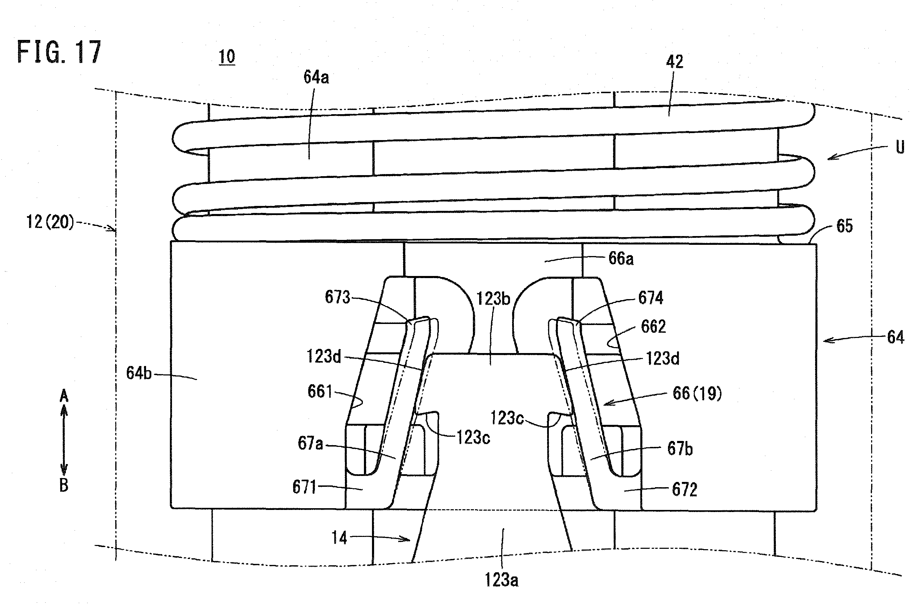

Inside the casing 12, the proximal end of the drive unit U moves toward the proximal end of the needle cover 14, and the first connections 66 and the second connections 123 face each other in the axial direction (directions of arrows A and B) (see Fig. 16). As illustrated in Fig. 17, when the drive unit U further moves in the distal direction (direction of arrow B), the projection 123b of each second connection 123 is inserted into the housing recess 66a of each first connection 66 from the distal end of the cylindrical portion 64b. In each housing recess 66a, the projection 123b is inserted between the first flexible arm portion 67a and the second flexible arm portion 67b (see Fig. 17).

As illustrated in Fig. 17, the bevels 123d of the projection 123b come into contact with the first flexible arm portion 67a and the second flexible arm portion 67b, whereby the first flexible arm portion 67a and the second flexible arm portion 67b are pressed in directions away from each other along the circumferential direction of the cylindrical portion 64b. Accordingly, the first and second flexible arm portions 67a and 67b tilt to be separated from each other, having the fixed ends 671 and 672 functioning as fulcrums.

The projection 123b and the extending end 123a are inserted into each housing recess 66a while the first and second flexible arm portions 67a and 67b are pushed out in the circumferential direction of the cylindrical portion 64b. As illustrated in Figs. 12 and 13, when the projection 123b passes over the free ends 673 and 674 of the first and second flexible arm portions 67a and 67b, the first flexible arm portion 67a and the second flexible arm portion 67b tilt by an elastic force in directions approaching each other (see the dot-dot-dash line in Fig. 17). Accordingly, the projection 123b of each second connection 123 is engaged with proximal ends of the first and second flexible arm portions 67a and 67b, and the free ends 673 and 674 of the first and second flexible arm portions 67a and 67b are engaged with the jaws 123c of the projection 123b. The first connections 66 of the sliding member 64 and the second connections 123 of the needle cover 14 are coupled in the axial direction (directions of arrows A and B) by each projection 123b, each first flexible arm portion 67a, and each second flexible arm portion 67b.

Hereinafter described are actions and effects of the medicinal solution administration device 10.

Let us first describe a case where the medicinal solution administration device 10 before use to which the cap 18 attached is accidentally dropped on the floor or the like from the cap 18 side (distal end).

When the medicinal solution administration device 10 is dropped and the cap 18 on the distal end touches the floor or the like, an impact (load) is applied to the cap 18 from the floor or the like. Along with this accident, the impact is transmitted from the cap 18 to the needle cover 14, which may cause the needle cover 14 to move relative to the casing 12 in the proximal direction (direction of arrow A) from a predetermined position (see the dot-dot-dash line in Fig. 4).

With the movement of the needle cover 14, distal portions of the engaging holes 120 and the outer hooks 150 of the retaining arms 148 come into contact with each other. With the contact between the distal portions of the engaging holes 120 and the outer hooks 150, the proximal ends of the retaining arms 148 including the outer hooks 150 are pressed radially outward. In this instance, the recesses 28 are not on the radially outer side of the proximal ends of the retaining arms 148, and the inner periphery of the cylinder 20 restricts the retaining arms 148 from tilting radially outward. The retaining arms 148 cannot tilt radially outward.

Accordingly, when the medicinal solution administration device 10 is dropped and an impact is applied to the needle cover 14, causing the needle cover 14 to move in the proximal direction, the engagement of the retaining arms 148 (outer hooks 150) with the engaging holes 120 are reliably maintained. With this configuration, the needle cover 14 is restricted from moving in the proximal direction relative to the casing 12.

In other words, when the cap 18 is attached, the retaining arms 148 (outer hooks 150) of the cap 18 function as a movement restricting mechanism 156 that restricts the movement of the needle cover 14 in the proximal direction relative to the casing 12.

When the medicinal solution administration device 10 is accidentally dropped and the cap 18 touches the floor or the like, an inertial force in the proximal direction acts on the sliding member 64 of the drive unit U. In this instance, as described above, the needle cover 14 is restricted from moving in the proximal direction relative to the casing 12, and the first connections 66 of the sliding member 64 and the second connections 123 of the needle cover 14 are coupled to each other. For this reason, the sliding member 64 is also restricted from moving in the proximal direction relative to the casing 12.

Therefore, even when the medicinal solution administration device 10 is accidentally dropped and an inertial force in the proximal direction acts on the needle cover 14, the coupling mechanism 19 reliably prevents the sliding member 64 from moving in the proximal direction. Accordingly, it is possible to reliably prevent erroneous actuation of the drive unit U attributed to an inertial force.

Hereinafter described is the medicinal solution administration by the medicinal solution administration device 10.

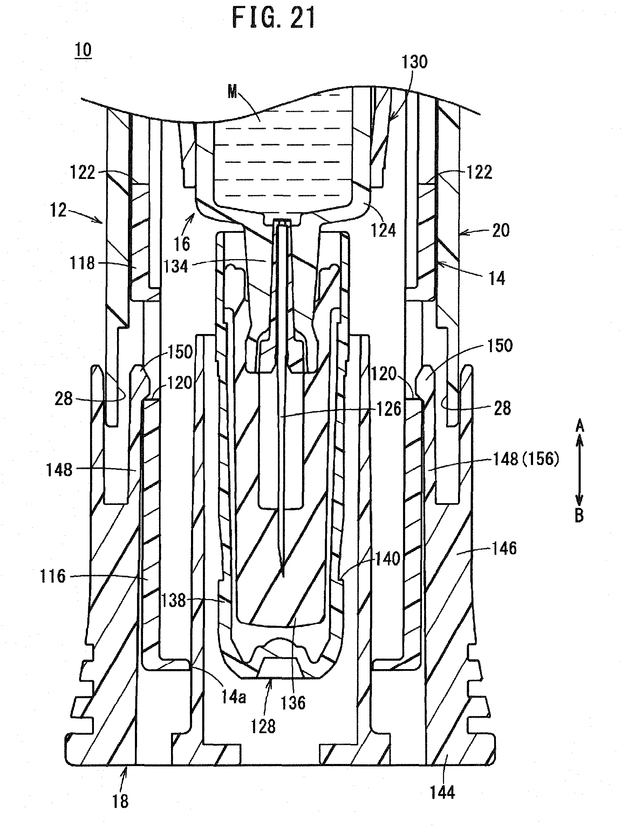

In the medicinal solution administration device 10 before use illustrated in Figs. 3 and 4, the cap 18 is detached from the distal ends of the casing 12 and the needle cover 14. In this instance, a patient holds the cylinder 20 of the casing 12 and pulls the cap 18 in a direction (direction of arrow B) in which the cap is separated from the casing 12. Accordingly, as illustrated in Fig. 21, the cap 18 moves in a direction away from the distal end of the needle cover 14, and the outer hooks 150 moves in the distal direction (direction of arrow B) inside the engaging holes 120.

With the movement of the outer hooks 150 in the distal direction, the outer hooks 150 are positioned to face the recesses 28. The cap 18 is further pulled in the distal direction (direction of arrow B). Accordingly, as illustrated in Fig. 22, the outer hooks 150 and the distal portions of the engaging holes 120 come into contact with each other, and the retaining arms 148 tilt radially outward. When the proximal ends of the retaining arms 148 having the outer hooks 150 move into the recesses 28, the engagement of the retaining arms 148 (outer hooks 150) with the engaging holes 120 is released. Then, the retaining arms 148 including the outer hooks 150 move in the distal direction through the recesses 28 and the gaps between the cylinder 20 and the needle cover 14.

With the movement of the cap 18 toward the distal end, the protective cover 128 retained by the inner hook 154 moves together with the cap 18. Accordingly, the protective cover 128 is detached from the needle retainer 134 of the barrel 124.

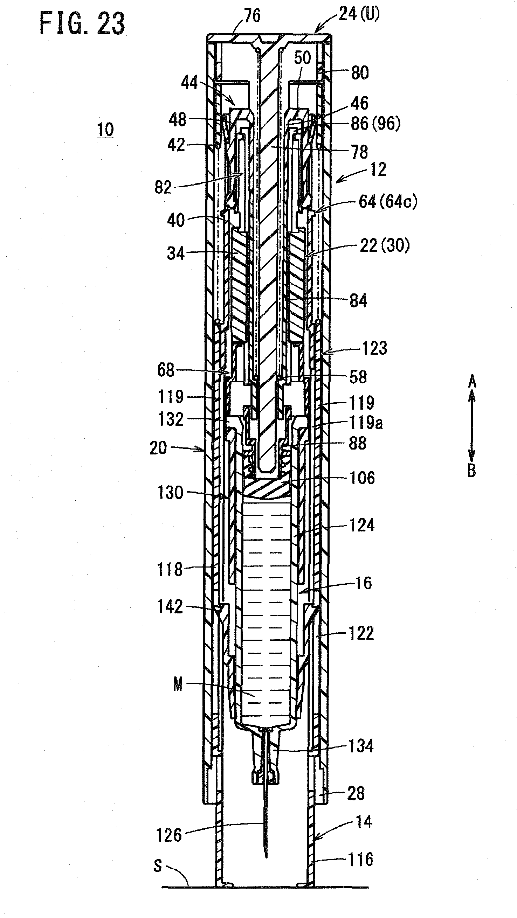

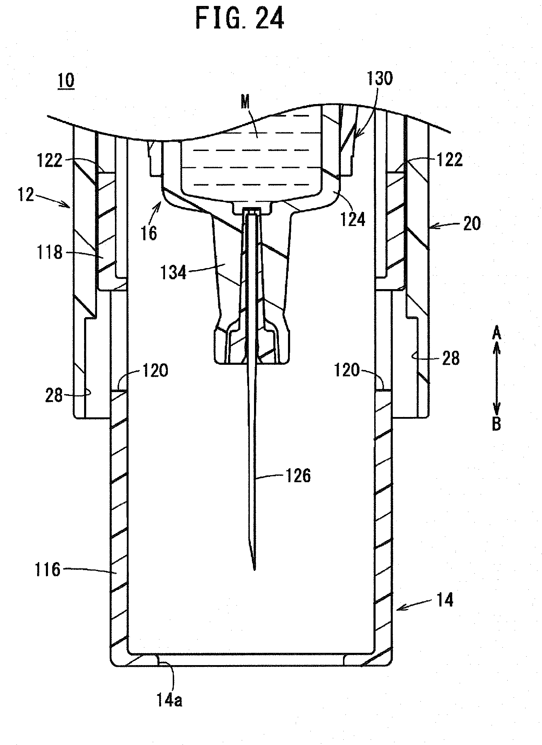

The engagement of the cap 18 with the needle cover 14 by the retaining arms 148 is released, and the cap 18 is completely detached from the distal end of the needle cover 14. Accordingly, as illustrated in Figs. 23 and 24, the distal ends of the casing 12 and the needle cover 14 are opened, and simultaneously, the protective cover 128 that has been covering the puncture needle 126 is detached.

Next, the medicinal solution M is administered using the medicinal solution administration device 10 from which the cap 18 is detached.

In a state before puncturing with the medicinal solution administration device 10, the relationship between the plunger 82 and the locking pin 44 is as illustrated in Fig. 7. The locking pin 44 is inserted into the interior of the flexible portions 86 of the plunger 82, and the flexible portions 86 are restricted from tilting inward by the locking pin 44. In other words, the flexible portions 86 cannot tilt inward. The brims 96 of the flexible portions 86 are engaged with the protrusions 38 of the sleeve body 22, which prevents the plunger 82 from moving in the distal direction.

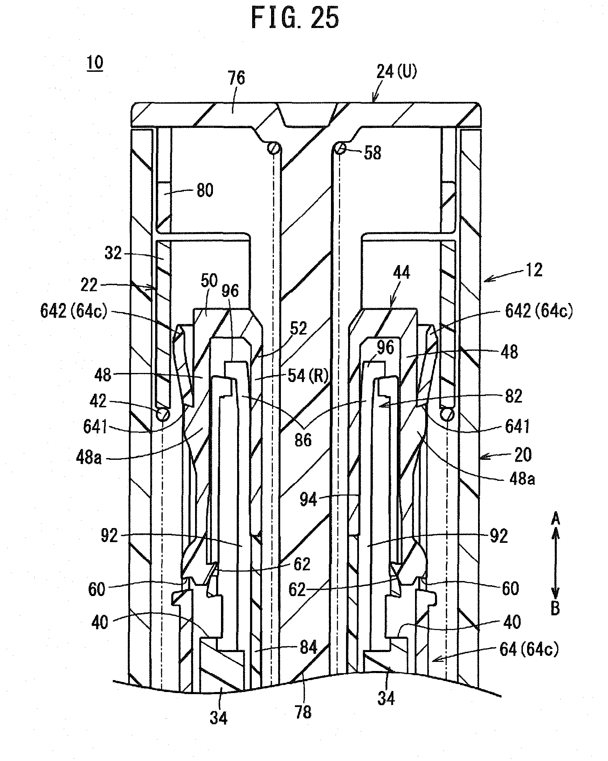

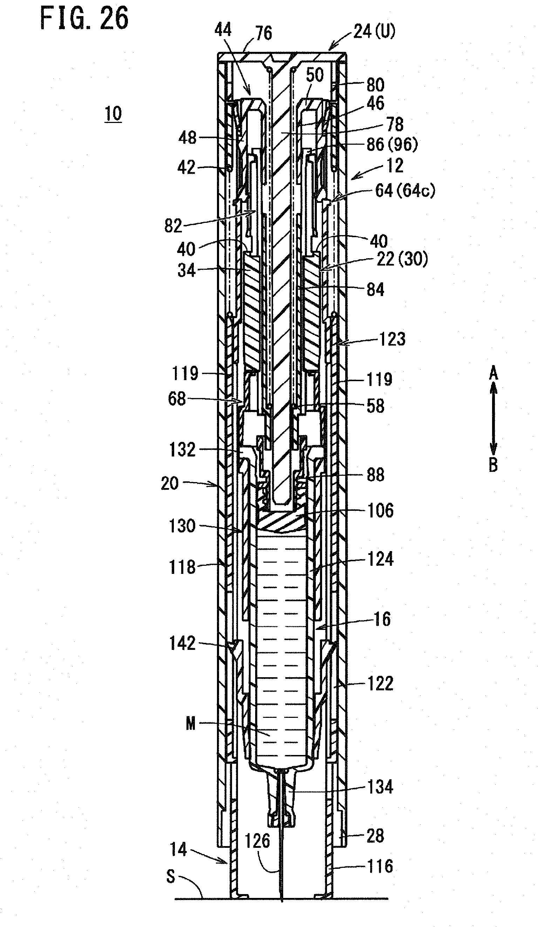

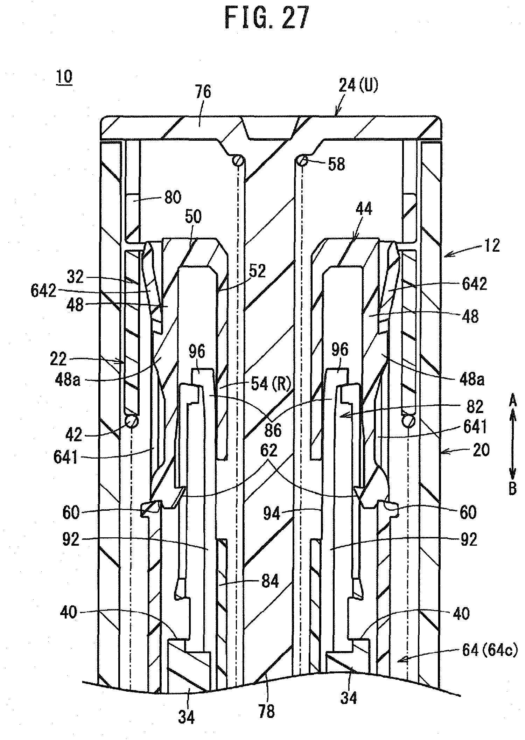

As illustrated in Fig. 23, the patient holds the casing 12 of the medicinal solution administration device 10 and presses the distal end of the needle cover 14 protruding from the distal end of the casing 12 against the skin S, or a desired puncture site, in such a manner that the distal end of the needle cover 14 is substantially perpendicular to the skin S. In this instance, the distal side and the proximal side of the medicinal solution administration device 10 are in states illustrated in Figs. 24 and 25, respectively. Next, as illustrated in Figs. 26 and 27, the casing 12 is continuously pushed against the skin S (distal side, direction of arrow B). With the casing 12 being pushed against the skin S, the skin S pushes the needle cover 14, whereby the needle cover 14 moves relative to the casing 12 in the proximal direction against an elastic force of the sleeve spring 42. Figs. 26 and 27 illustrate a state where a needle tip of the puncture needle 126 has just started to touch the skin S.

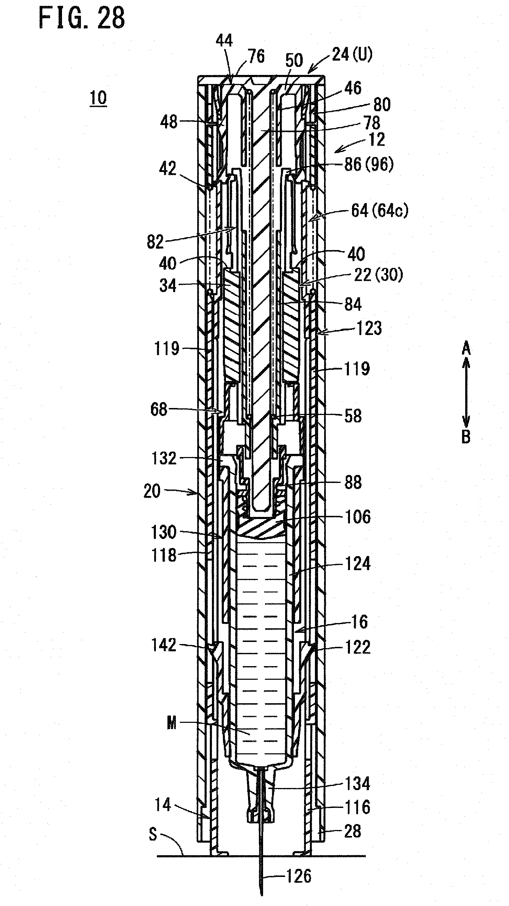

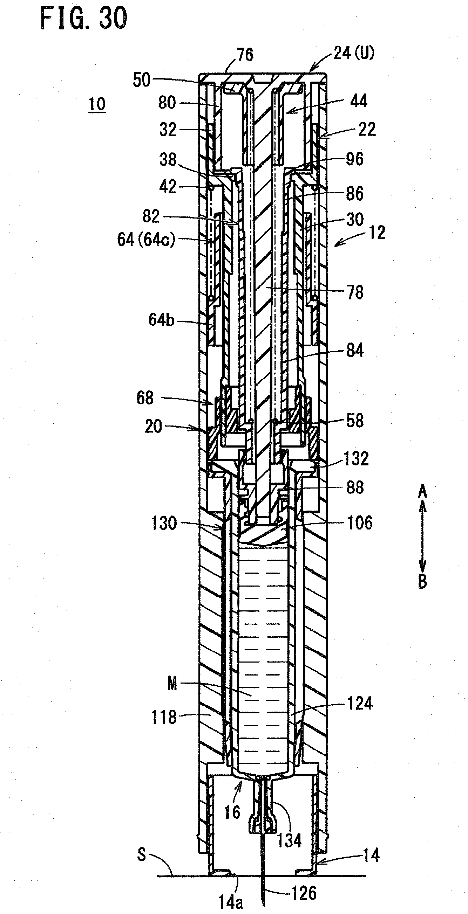

With the casing 12 further pushed against the skin S, the puncture needle 126 of the syringe 16 protrudes from the hole 14a of the needle cover 14 toward the distal side (direction of arrow B) as illustrated in Figs. 28 and 30. Accordingly, the state is changed to a puncture completed state where the puncture needle 126 pierces the skin S and is inserted to a predetermined depth. In the puncture completed state, the distal end of the cylinder 20 is substantially at the same position as the distal end of the needle cover 14.

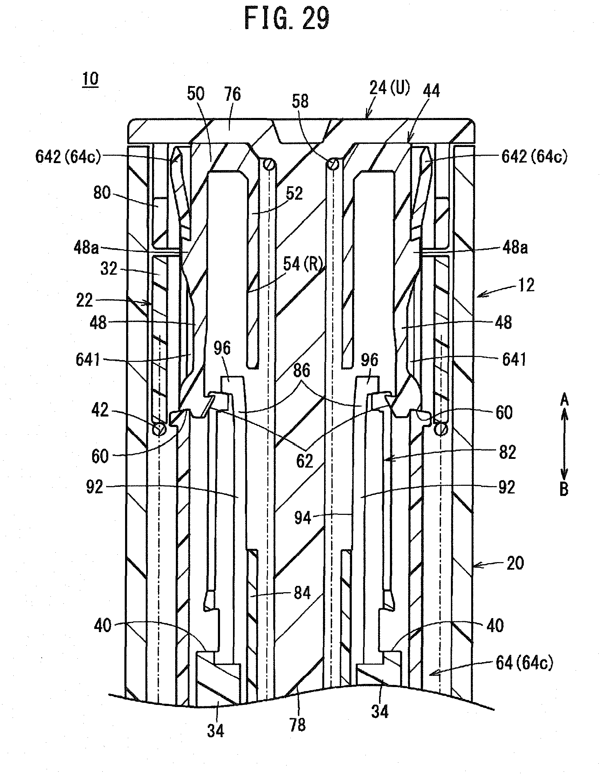

The ribs 119a of the needle cover 14 are in contact with the distal end of the sliding member 64. Therefore, in the process of state change from Fig. 23 to Fig. 28, along with the movement of the needle cover 14 in the proximal direction relative to the casing 12 as described above, the sliding member 64 moves in the proximal direction relative to the casing 12. As illustrated in Figs. 27 and 29, with the movement of the sliding member 64 in the proximal direction, the distal ends of the slits 641 of the sliding member 64 push the engaging ends 60 disposed on the distal ends of the arms 48 of the locking pin 44 in the proximal direction, whereby the locking pin 44 also moves in the proximal direction relative to the casing 12.

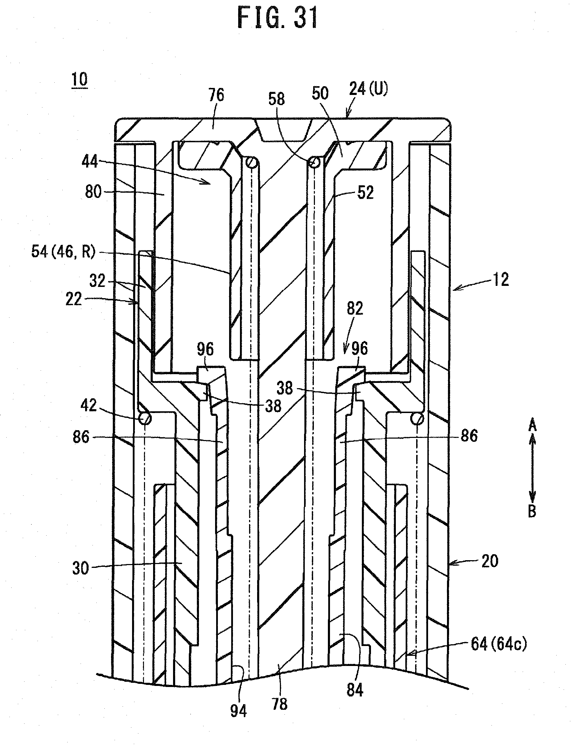

With the movement of the locking pin 44 in the proximal direction (direction of arrow A) relative to the casing 12, the small diameter portion 54 of the locking pin 44 is separated from the interior of the flexible portions 86 of the plunger 82 in the proximal direction (direction of arrow A) as illustrated in Fig. 31. Accordingly, the locked state of the plunger 82 by the locking pin 44 is released, and the plunger 82 starts moving in the distal direction (direction of arrow B) by an elastic force of the injection spring 58.

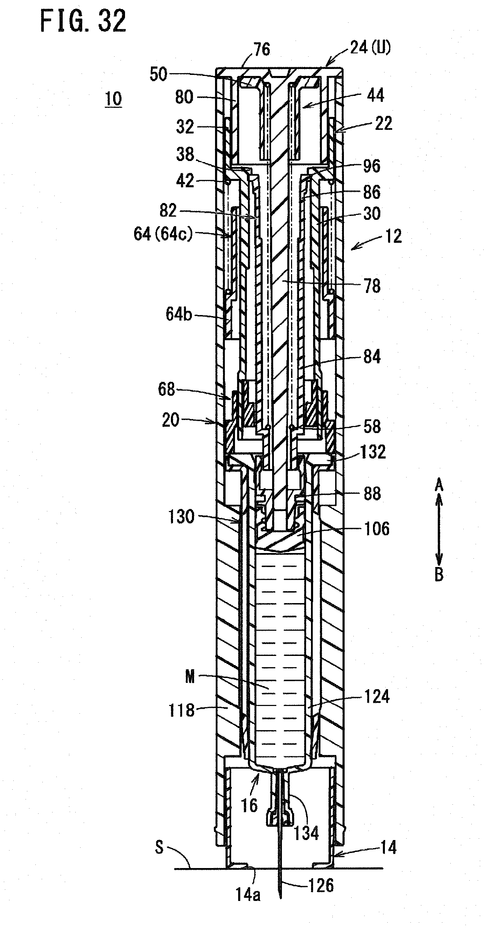

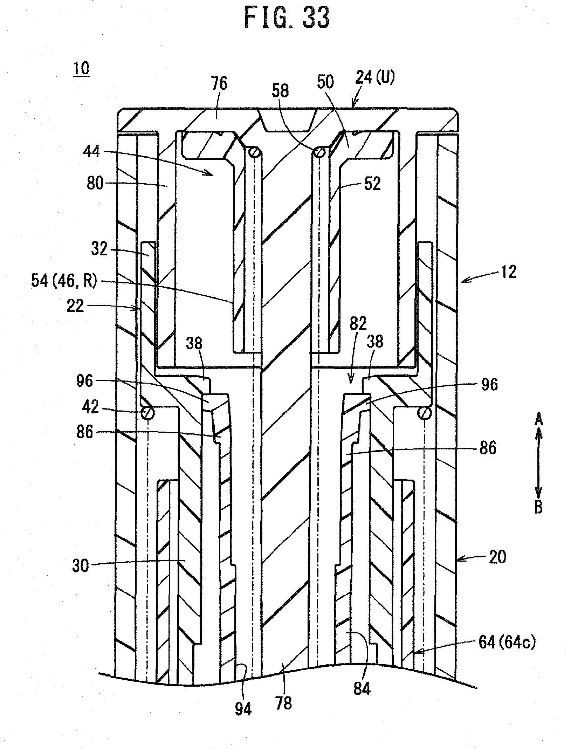

When the plunger 82 starts moving in the distal direction from the state illustrated in Fig. 31, the four brims 96 are pushed radially inward by the contact between the tapered distal surfaces and the protrusions 38. Accordingly, the flexible portions 86 tilt radially inward from the pin main structure 46. As illustrated in Figs. 32 and 33, when the plunger 82 further moves in the distal direction, the brims 96 pass over the protrusions 38 in the distal direction.

When the brims 96 of the plunger 82 pass over the protrusions 38, the brims 96 are displaced radially inward, and then, expand radially outward again by an elastic restoring force of the flexible portions 86, and outer edge portions come into contact with the inner periphery of the body main structure 30. The outside diameter D1 of the brims 96 is slightly larger than the inside diameter D2 of the body main structure 30 (see Fig. 7). Accordingly, when the brims 96 come into contact with the body main structure 30, a first notification sound, or a sound of contact (click), is generated.

After the plunger 82 starts moving in the distal direction and the brims 96 pass over the protrusions 38, the plunger 82 further moves in the distal direction, and the plunger 82 starts pressing the gasket 106. The medicinal solution M in the barrel 124 is pressed toward the distal end by the gasket 106. Accordingly, the medicinal solution M is ejected from the puncture needle 126, and the medicinal solution M is subcutaneously administered to the patient. As described above, when the plunger 82 starts moving in the distal direction and the brims 96 pass over the protrusions 38, the first notification sound is generated due to the brims 96 tapping the body main structure 30. The first notification sound enables the patient to recognize the start of administration of the medicinal solution M.

When the plunger 82 moves in the distal direction, the plunger 82 is introduced by each guiding rib 34 of the sleeve body 22 inserted into the four sliding grooves 92. Accordingly, when the plunger 82 moves toward the distal end, the plunger 82 moves along the axial direction without rotating. The gasket 106 attached to the top 88 is inserted into the barrel 124 of the syringe 16.

After the administration of the medicinal solution M is started, the plunger 82 continuously moves in the distal direction (direction of arrow B) at a constant speed by an elastic force of the injection spring 58. The medicinal solution M is pushed out by the gasket 106 moving in the distal direction inside the barrel 124, and the medicinal solution M is ejected from the puncture needle 126. The plunger 82 further moves in the distal direction, and the flexible portions 86 reach a proximal end of the end guide 68.

As illustrated in Fig. 18A, the brims 96 come into contact with the inclined guiding portions 72, and the brims 96 move in the distal direction (direction of arrow B) while rotating clockwise along the inclined guiding portions 72. In other words, the movement of the plunger 82 along the inclined guiding portions 72 of the end guide 68 applies a rotational force to the plunger 82.

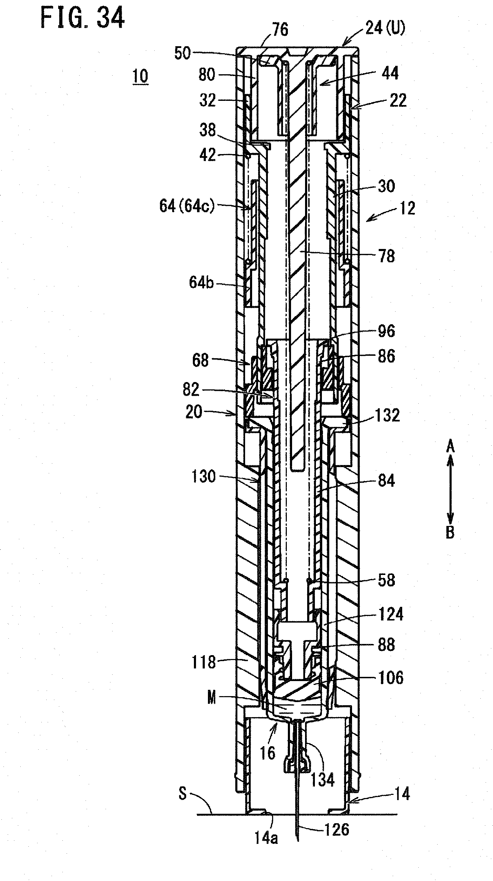

Simultaneously, as illustrated in Fig. 18B, the rotation of the plunger 82 causes the engaging chips 98 disposed on its distal ends to move along the horizontal portions 110 in the introducing grooves 108 of the top 88. As illustrated in Fig. 34, the plunger 82 reaches the distal ends of the guiding ribs 34 of the sleeve body 22, and the plunger 82 which is guided in the axial direction by the sliding grooves 92 and the guiding ribs 34 is released and becomes rotatable.

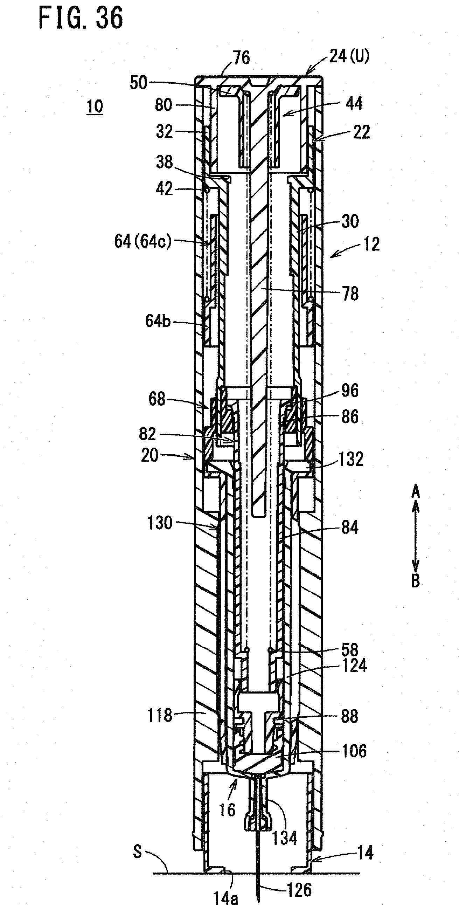

As illustrated in Fig. 19A, the flexible portions 86 move in the distal direction while rotating along the inclined guiding portions 72 and reach the linear guiding portions 74. In this instance, as illustrated in Fig. 36, the plunger 82 administers a predetermined amount of the medicinal solution M in the barrel 124 to the skin S through the puncture needle 126, thereby completing the administration of the medicinal solution M.

Before completion of administration of the medicinal solution M, as illustrated in Fig. 19B, the engaging chips 98 of the rotating plunger 82 reach the inclined portions 114 of the introducing grooves 108. The guiding grooves 70 function as a guide unit that linearly moves the plunger 82 in the distal direction (direction of arrow B) after rotating the plunger 82 at a predetermined degree.

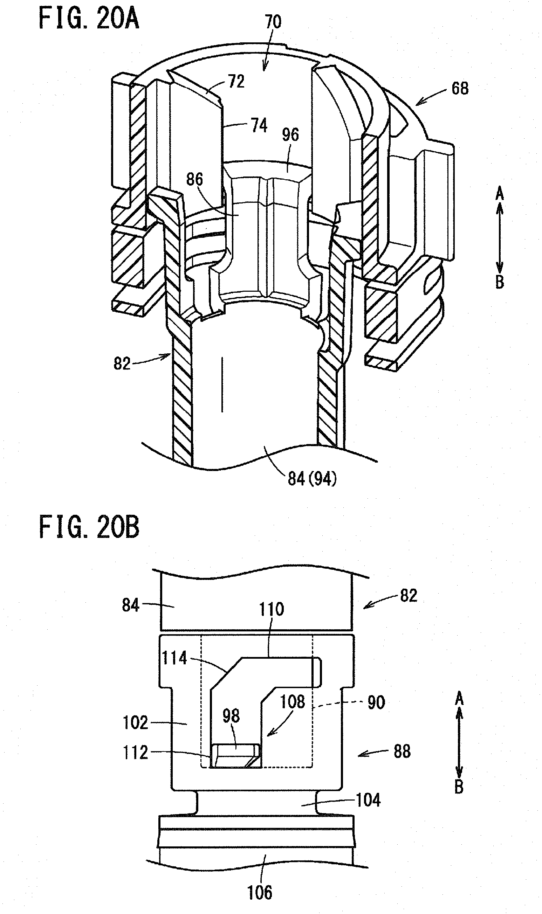

After completion of administration of the medicinal solution M, the engaging chips 98 of the plunger 82 move toward the perpendicular portions 112 while rotating along the inclined portions 114. The engaging chips 98 of the plunger 82 move along the perpendicular portions 112, thereby moving again in the axial direction toward the distal direction (direction of arrow B). As illustrated in Fig. 20B, distal surfaces of the engaging chips 98 moved along the perpendicular portions 112 of the introducing grooves 108 come into contact with distal edges of the perpendicular portions 112, which causes a second notification sound, or a sound of contact (click).

After completion of administration of the medicinal solution M, the second notification sound is generated a predetermined time later. The second notification sound enables the patient to recognize the completion of medicinal solution administration to the affected area (skin S). After the patient recognizes the second notification sound, the patient separates the medicinal solution administration device 10 from the affected area.

In other words, after the completion of medicinal solution administration by the medicinal solution administration device 10, the plunger 82 is rotated by the guiding grooves 70 of the end guide 68 to decelerate the moving speed in the axial direction. This delays the time for the gasket 106 of the top 88 connected to the plunger 82 to reach the distal end of the barrel 124. After completion of medicinal solution administration, the second notification sound is generated a predetermined time later to notify the completion of medicinal solution administration.

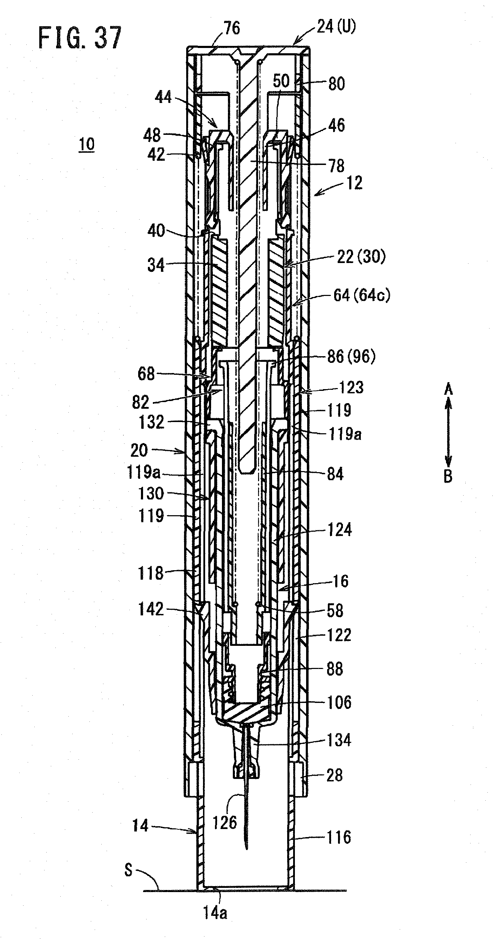

When the patient releases a pressing force of the medicinal solution administration device 10 against the skin S, the sliding member 64 is biased toward the distal end by an elastic force of the sleeve spring 42 as illustrated in Fig. 37. The distal end of the sliding member 64 is in contact with the ribs 119a (see Fig. 11) of the needle cover 14, whereby the sliding member 64 pushes the needle cover 14 in the distal direction and moves in the distal direction relative to the casing 12 together with the needle cover 14. The needle cover 14 moves to a position where the distal end of the needle cover 14 moves further in the distal direction than the puncture needle 126. Accordingly, the puncture needle 126 is completely covered by the needle cover 14.

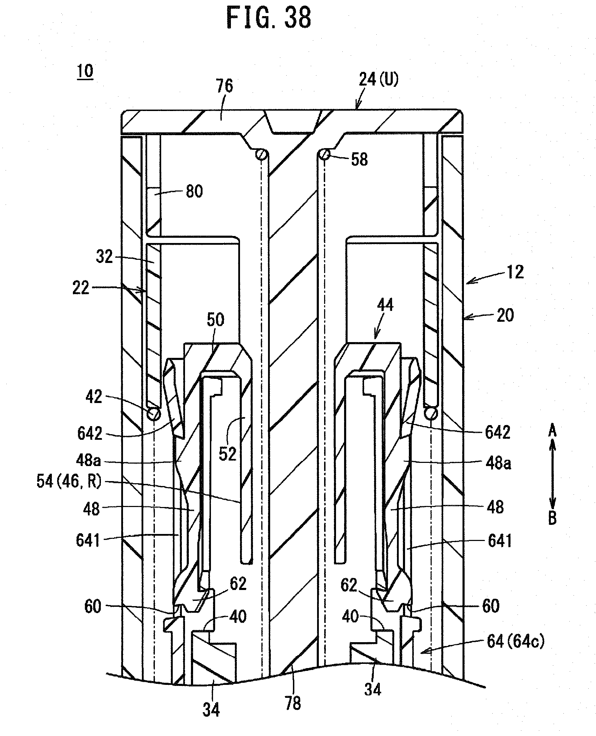

As illustrated in Fig. 38, the protruding pieces 642 of the sliding member 64 are engaged with the protrusive portions 48a of the locking pin 44, and the locking pin 44 moves in the distal direction (direction of arrow B) together with the sliding member 64. With the movement of the locking pin 44 in the distal direction, the locking claws 62 are engaged with the locking grooves 40 of the sleeve body 22. Accordingly, the movement of the locking pin 44 in the proximal direction relative to the sleeve body 22 is restricted. The sliding member 64 abuts on the arms 48 of the locking pin 44, and the movement of the sliding member 64 in the proximal direction is also restricted. The extending portions 119 (ribs 119a) of the needle cover 14 abut on the distal end of the sliding member 64, and the movement of the needle cover 14 in the proximal direction is also restricted.

The needle cover 14 is restricted from moving in the proximal direction. For this reason, even when a force in the proximal direction acts on the needle cover 14, the puncture needle 126 is secured and not exposed to the outside from the needle cover 14.

As described above, in this embodiment, the drive unit U is capable of biasing the gasket 106 of the syringe 16 toward the distal end by the plunger 82 along with the movement of the needle cover 14 in the proximal direction, and the proximal end of the needle cover 14 and the drive unit U are connected to each other in the axial direction by the coupling mechanism 19. Therefore, when the medicinal solution administration device 10 is dropped from the cap 18 side, the movement restricting mechanism 156 prevents the needle cover 14 from moving in the proximal direction relative to the cap 18, and when an inertial force acts on the drive unit U in the proximal direction, the drive unit U (sliding member 64) coupled to the needle cover 14 by the coupling mechanism 19 does not move in the proximal direction.

Accordingly, even when the medicinal solution administration device 10 is dropped, the needle cover 14 is reliably prevented from moving in the proximal direction, and the drive unit U connected to the needle cover 14 is prevented from moving in the proximal direction, which prevents the erroneous actuation of the drive unit U.

The proximal end of the needle cover 14 can be reliably coupled to the distal end of the drive unit U in the axial direction by the first connections 66 of the drive unit U and the second connections 123 of the needle cover 14. Therefore, when the needle cover 14 is biased in the proximal direction by inertia at the time of dropping the medicinal solution administration device 10, the needle cover 14 is reliably prevented from moving in the proximal direction (direction of arrow A).

The drive unit U includes the sliding member 64 that is movable along the axial direction of the casing 12 and the sleeve spring 42 that biases the sliding member 64 toward the syringe 16, and the first connections 66 are disposed on the sliding member 64. Accordingly, coupling the second connections 123 of the needle cover 14 to the first connections 66 disposed on the sliding member 64 makes it possible to couple the needle cover 14 to the sliding member 64 reliably, thereby preventing the sliding member 64 from moving in the proximal direction (direction of arrow A) when the medicinal solution administration device 10 is dropped.

When assembling the medicinal solution administration device 10, the first and second flexible arm portions 67a and 67b of each first connection 123 are elastically deformed by the projection 123b of each second connection 66 protruding in the direction perpendicular to the axial direction of the casing 12, and the projection 123b is engaged with the first and second flexible arm portions 67a and 67b. Accordingly, the first connections 66 and the second connections 123 are coupled to each other, which reliably prevents the sliding member 64 from moving in the proximal direction (direction of arrow A).

The proximal end of the needle cover 14 is provided with the extending portions 119 protruding toward the proximal end, the projection 123b of each second connection 123 is disposed on the proximal end of each extending portion 119, and the projection 123b protrudes toward both sides of each extending portion 119 in the width direction perpendicular to the direction of extension of the extending portions 119. Therefore, the needle cover 14 can be coupled to the first connections 66 of the drive unit U at the most proximal side of the needle cover 14, and the needle cover 14 and the drive unit U can be reliably coupled at an initial stage of assembly of the needle cover 14 and the drive unit U.

When assembling the needle cover 14 and the drive unit U, the second connections 123 can be connected to the first connections 66 more firmly by engaging the projection 123b of each second connection 123 protruding toward both sides in the width direction perpendicular to the axial direction, or a direction of assembly, with each first connection 66.

The first flexible arm portion 67a and the second flexible arm portion 67b of each first connection 66 are arranged in pairs, being separated from each other along the circumferential direction of the cylindrical portion 64b disposed on the proximal end of the sliding member 64, and the first flexible arm portion 67a and the second flexible arm portion 67b have a cantilever structure in which the distal ends of the first flexible arm portion 67a and the second flexible arm portion 67b are connected to the distal end of the cylindrical portion 64b.

With this configuration, when the extending portion 119 of each second connection 123 is inserted between the pair of first flexible arm portion 67a and the second flexible arm portion 67b, the first flexible arm portion 67a and the second flexible arm portion 67b are elastically deformed by the projection 123b in directions away from each other along the circumferential direction of the cylindrical portion 64b with the distal ends functioning as fulcrums, and when the projection 123b reaches the proximal ends of the first and second flexible arm portions 67a and 67b, the projection 123b is engaged with the proximal ends.

Accordingly, the projection 123b is engaged with the proximal ends of the pair of first and second flexible arm portions 67a and 67b, and the first and second flexible arm portions 67a and 67b and the projection 123b are coupled in the axial direction, thereby preventing the movement of the sliding member 64 in the proximal direction (direction of arrow A).

The needle cover 14 includes the ribs 119a protruding in a direction opposite to the casing 12 and locked by the distal end of the sliding member 64. Accordingly, when the needle cover 14 is moved in the proximal direction (direction of arrow A) due to the distal end of the needle cover 14 being pressed against the skin S, the ribs 119a come into contact with the distal end of the sliding member 64 and the sliding member 64 is pushed up in the proximal direction by the needle cover 14, which allows the drive unit U to actuate.

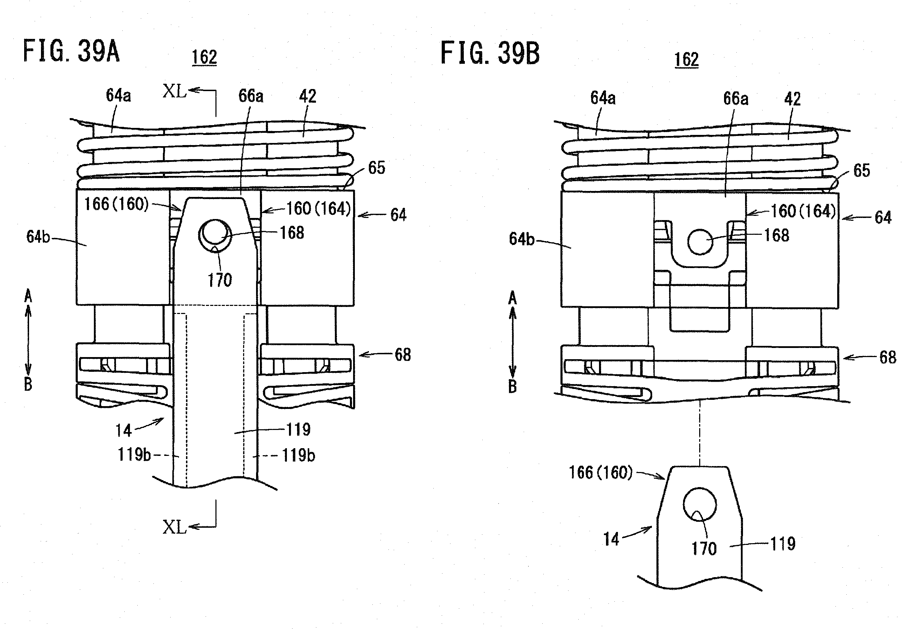

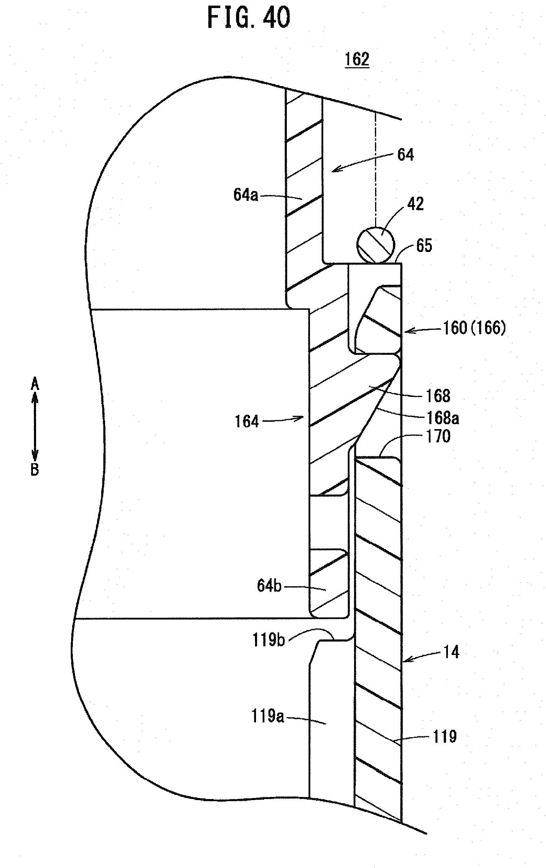

For example, a coupling mechanism 160 according to a modification illustrated in Figs. 39A to 40 may be employed in a medicinal solution administration device 162. The coupling mechanism 160 includes a first connection 164 disposed in a cylindrical portion 64b of a sliding member 64 and a second connection 166 disposed on a proximal end of a needle cover 14.

The first connection 164 includes a protrusion 168 on the inner periphery of a housing recess 66a. The protrusion 168 has a circular shape and protrudes radially outward from the inner periphery of the housing recess 66a. A top of the protrusion 168 has a bevel 168a extending radially outward from a proximal end toward a distal end of the sliding member 64.

The second connection 166 has an insertion hole (hole) 170 on a proximal end of an extending portion 119 in the needle cover 14. The insertion hole 170 has a circular shape. The insertion hole 170 penetrates the extending portion 119 and allows insertion of the protrusion 168 of the first connection 164.

When assembling the medicinal solution administration device 162, the protrusion 168 of the first connection 164 in a drive unit U (sliding member 64) is inserted into the insertion hole 170 of the second connection 166 of the needle cover 14, whereby the first connection 164 of the sliding member 64 and the second connection 166 of the needle cover 14 are coupled to each other in the axial direction. Accordingly, when the medicinal solution administration device 162 is dropped and a load is applied to a distal end, the protrusion 168 reliably prevents the sliding member 64 from moving in the proximal direction (direction of arrow A).

When the bevel 168a of the protrusion 168 moves along the inner surface of the extending portion 119 that has the insertion hole 170, the extending portion 119 tilts gradually and radially outward while being in contact with the bevel 168a, and when the protrusion 168 and the insertion hole 170 face each other, the protrusion 168 is easily and reliably inserted into the insertion hole 170.

The following description summarizes the embodiment.

According to the embodiment, a medicinal solution administration device (10, 162) includes:
a casing (12) formed into a hollow cylindrical shape;
a syringe (16) including a cylinder (20) that is housed in the casing and filled with a medicinal solution (M) and a puncture needle (126) that is communicated with the cylinder and administers the medicinal solution to a living body;
a needle cover (14) having a hollow cylindrical shape that is disposed inside the casing and covers a distal side of the syringe and displaces in a proximal direction relative to the casing when being pressed against a puncture target;
a cap (18) that is detachably disposed on a distal end of the needle cover and detached when the puncture target is punctured with the puncture needle; and
a drive unit (U) that is housed on a proximal side of the casing and biases a gasket (106) of the syringe toward a distal end of the casing by a plunger (82) along with movement of the needle cover in the proximal direction, wherein
the drive unit includes a locking mechanism (R) that blocks the plunger from moving in a distal direction in an initial state and allows the plunger to move in the distal direction along with the movement of the needle cover in the proximal direction, and
the medicinal solution administration device includes
a movement restricting mechanism (156) that restricts the movement of the needle cover in the proximal direction relative to the casing when the cap is attached and allows the movement of the needle cover in the proximal direction when the cap is detached from the casing and
a coupling mechanism (19, 160) that couples a proximal end of the needle cover and the drive unit to each other in an axial direction.

The coupling mechanism includes
a first connection (66, 164) disposed in the drive unit and
a second connection (123, 166) disposed on the proximal end of the needle cover and coupled to the first connection.

The drive unit includes
a sliding member (64) movable along the axial direction of the casing and
an elastic member (42) that biases the sliding member toward the syringe, and
the first connection is disposed on the sliding member.

The second connection (123) has a projection (123b) protruding in a direction perpendicular to the axial direction of the casing, and the first connection (66) has a flexible arm portion (67a, 67b) engaged with the projection and elastically deformable by contact with the projection.

The second connection (166) has a hole (170) opened in a direction perpendicular to the axial direction of the casing, and the first connection (164) has a protrusion (168) protruding toward the second connection and engaged with the hole.

The proximal end of the needle cover is provided with an extending portion (119) further protruding toward the proximal end, the projection of the second connection is disposed on a proximal end of the extending portion, and
the projection protrudes toward both sides of the extending portion in a width direction perpendicular to a direction of extension of the extending portion.

The sliding member has a proximal end including a cylindrical portion (64b) whose distal end is provided with the first connection, and
the second connection is provided with a pair of flexible arm portions separated from each other along a circumferential direction of the cylindrical portion, the flexible arm portions having a cantilever structure in which distal ends of the flexible arm portions are connected to the distal end of the cylindrical portion, the flexible arm portions being inclined from the distal ends toward proximal ends of the flexible arm portions, and one flexible arm portion and the other flexible arm portion approaching each other toward the proximal ends.

The needle cover includes a rib (119a) protruding in a direction opposite to the casing and locked by a distal end of the sliding member.

Note that the present invention is not limited to the disclosure, and various configurations may be adopted without departing from the gist of the present invention.

## Claims

1. A medicinal solution administration device comprising:
a casing formed into a hollow cylindrical shape;
a syringe including a cylinder that is housed in the casing and filled with a medicinal solution and a puncture needle that is communicated with the cylinder and administers the medicinal solution to a living body;
a needle cover having a hollow cylindrical shape that is disposed inside the casing and covers a distal side of the syringe and displaces in a proximal direction relative to the casing when being pressed against a puncture target;
a cap that is detachably disposed on a distal end of the needle cover and detached when the puncture target is punctured with the puncture needle; and
a drive unit that is housed on a proximal side of the casing and biases a gasket of the syringe toward a distal end of the casing by a plunger along with movement of the needle cover in the proximal direction, wherein
the drive unit includes a locking mechanism that blocks the plunger from moving in a distal direction in an initial state and allows the plunger to move in the distal direction along with the movement of the needle cover in the proximal direction, and
the medicinal solution administration device includes a movement restricting mechanism that restricts the movement of the needle cover in the proximal direction relative to the casing when the cap is attached and allows the movement of the needle cover in the proximal direction when the cap is detached from the casing and
a coupling mechanism that couples a proximal end of the needle cover and the drive unit to each other in an axial direction.

2. The medicinal solution administration device according to claim 1, wherein
the coupling mechanism includes
a first connection disposed in the drive unit and
a second connection disposed on the proximal end of the needle cover and coupled to the first connection.

3. The medicinal solution administration device according to claim 2, wherein
the drive unit includes
a sliding member movable along the axial direction of the casing and
an elastic member that biases the sliding member toward the syringe, and
the first connection is disposed on the sliding member.

4. The medicinal solution administration device according to claim 2 or 3, wherein
the second connection has a projection protruding in a direction perpendicular to the axial direction of the casing, and the first connection has a flexible arm portion engaged with the projection and elastically deformable by contact with the projection.

5. The medicinal solution administration device according to claim 2 or 3, wherein
the second connection has a hole opened in a direction perpendicular to the axial direction of the casing, and the first connection has a protrusion protruding toward the second connection and engaged with the hole.

6. The medicinal solution administration device according to claim 4, wherein
the proximal end of the needle cover is provided with an extending portion further protruding toward the proximal end, the projection of the second connection is disposed on a proximal end of the extending portion, and
the projection protrudes toward both sides of the extending portion in a width direction perpendicular to a direction of extension of the extending portion.

7. The medicinal solution administration device according to claim 3, wherein
the sliding member has a proximal end including a cylindrical portion whose distal end is provided with the first connection, and
the second connection is provided with a pair of flexible arm portions separated from each other along a circumferential direction of the cylindrical portion, the flexible arm portions having a cantilever structure in which distal ends of the flexible arm portions are connected to the distal end of the cylindrical portion, the flexible arm portions being inclined from the distal ends toward proximal ends of the flexible arm portions, and one flexible arm portion and the other flexible arm portion approaching each other toward the proximal ends.

8. The medicinal solution administration device according to claim 7, wherein
the needle cover includes a rib protruding in a direction opposite to the casing and locked by a distal end of the sliding member.
